(19) Europäisches Patentamt
European Patent Office
Office européen des brevets

(11) EP 4 740 836 A1

(12) EUROPEAN PATENT APPLICATION

(43) Date of publication:
**13.05.2026 Bulletin 2026/20**

(21) Application number: **25211656.1**

(22) Date of filing: **28.10.2025**

(51) International Patent Classification (IPC):
***A61B 3/12*** (2006.01) ***A61B 3/14*** (2006.01)
***A61B 3/15*** (2006.01)

(52) Cooperative Patent Classification (CPC):
**A61B 3/156; A61B 3/12; A61B 3/14**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **06.11.2024 JP 2024194444**

(71) Applicant: **Topcon Corporation**
**Tokyo 174-8580 (JP)**

(72) Inventors:
- **TOMITA, Sora**
**Tokyo, 174-8580 (JP)**
- **NAKAJIMA, Masashi**
**Tokyo, 174-8580 (JP)**
- **SUZUKI, Masaya**
**Tokyo, 174-8580 (JP)**

(74) Representative: **Gagel, Roland**
**Patentanwalt Dr. Roland Gagel**
**Landsberger Strasse 480a**
**81241 München (DE)**

# (54) OPHTHALMIC APPARATUS

(57) An ophthalmic apparatus includes an objective lens, an illumination optical system, an optical scanner, a light receiving optical system, and a pupil division member. The illumination optical system includes an illumination diaphragm with one or more openings formed at positions eccentric to an optical axis, and is configured to irradiate slit-shaped illumination light. The optical scanner is configured to deflect the illumination light to guide the deflected illumination light to the eye to be examined via the objective lens. The light receiving optical system is configured to guide returning light, that passes through the objective lens, of the illumination light from the eye to be examined to an image sensor. The pupil division member includes a photographic diaphragm with one or more openings formed at positions eccentric to an optical axis, and is configured to spatially separate optical paths of the illumination optical system and the light receiving optical system.

EP 4 740 836 A1

## Description

FIELD

The disclosure relates to an ophthalmic apparatus.

BACKGROUND

In recent years, screening tests have been performed using ophthalmic apparatuses. Such ophthalmic apparatuses are expected to be applied to self-examinations, and further downsizing and weight saving of the ophthalmic apparatuses are desired.

U.S. Patent No. 7,831,106, and U.S. Patent No. 8,237,835 disclose ophthalmic apparatuses configured to pattern-illuminate a fundus of an eye to be examined using slit-shaped light and to detect returning light thereof using an image sensor. These ophthalmic apparatuses can acquire clear images of the fundus of the eye to be examined with a simple configuration, by adjusting the illumination pattern and the timing of light receiving using the image sensor.

In such ophthalmic apparatuses, illumination light must be incident into the eye through a pupil of the eye to be examined, and reflected light (returning light, fundus reflected light) of the illumination light from the fundus must be emitted through the pupil. Therefore, in the ophthalmic apparatuses, an image of an illumination opening, through which the illumination light passes, and an image of a light receiving opening (photographing opening), through which the reflected light passes, are separated on a pupil conjugate plane at a position substantially conjugate optically to the pupil of the eye to be examined.

WO 2021/205965 discloses an ophthalmic apparatus configured to illuminate the fundus with slit-shaped illumination light passing through two illumination openings and to receive returning light, which passes through a single light receiving opening, from the fundus. In this ophthalmic apparatus, the slit-shaped illumination light is generated by irradiating the illumination light onto a slit formed so as to suppress occurrence of flare.

Japanese Unexamined Patent Application Publication No. 2020-06172 discloses an ophthalmic apparatus configured to fit an image of an illumination opening and an image of a light receiving opening within the pupil of the eye to be photographed, by shifting an alignment reference position relative to an eye to be examined in accordance with a size of a pupil diameter.

SUMMARY

One aspect of some embodiments is an ophthalmic apparatus including: an objective lens; an illumination optical system including an illumination diaphragm with one or more openings formed at positions eccentric to an optical axis, the illumination diaphragm being arranged at a position substantially conjugate optically to a pupil of an eye to be examined, and configured to irradiate slit-shaped illumination light; an optical scanner arranged at a position substantially conjugate optically to the pupil, and configured to deflect the illumination light to guide the deflected illumination light to the eye to be examined via the objective lens; a light receiving optical system configured to guide returning light of the illumination light from the eye to be examined to an image sensor, the returning light passing through the objective lens, the image sensor being arranged at a position substantially conjugate optically to a photographed site of the eye to be examined; and a pupil division member including a photographic diaphragm with one or more openings formed at positions eccentric to an optical axis, the photographic diaphragm being arranged at a position substantially conjugate optically to the pupil, and configured to spatially separate an optical path of the illumination optical system and an optical path of the light receiving optical system.

BRIEF DESCRIPTION OF THE DRAWINGS

FIG. 1 is a schematic diagram illustrating an example of a configuration of an optical system of an ophthalmic apparatus according to embodiments.

FIG. 2 is a schematic diagram illustrating an example of a configuration of an optical system of the ophthalmic apparatus according to the embodiments.

FIG. 3 is a schematic diagram illustrating an example of the configuration of the optical system of the ophthalmic apparatus according to the embodiments.

FIG. 4 is a schematic diagram for explaining the configuration of the optical system of the ophthalmic apparatus according to the embodiments.

FIG. 5 is an explanatory diagram of an operation of the ophthalmic apparatus according to the embodiments.

FIG. 6 is an explanatory diagram of an operation of the ophthalmic apparatus according to the embodiments.
FIG. 7 is an explanatory diagram of an operation of the ophthalmic apparatus according to the embodiments.
FIG. 8 is a schematic diagram for explaining a configuration of the optical system of the ophthalmic apparatus according to the embodiments.
FIG. 9 is a schematic diagram for explaining a configuration of the optical system of the ophthalmic apparatus according to the embodiments.
FIG. 10 is a schematic diagram for explaining a configuration of the optical system of the ophthalmic apparatus according to the embodiments.
FIG. 11 is a schematic diagram illustrating an example of a configuration of a processing system of the ophthalmic apparatus according to the embodiments.
FIG. 12 is a flowchart illustrating an example of an operation of the ophthalmic apparatus according to the embodiments.
FIG. 13A is an explanatory diagram of the operation of the ophthalmic apparatus according to the embodiments.
FIG. 13B is an explanatory diagram of the operation of the ophthalmic apparatus according to the embodiments.
FIG. 14A is a schematic diagram illustrating an example of a configuration of an optical system of an ophthalmic apparatus according to a first modification example of the embodiments.
FIG. 14B is a schematic diagram for explaining a configuration of an optical system of the ophthalmic apparatus according the first modification example of the embodiments.
FIG. 15 is a schematic diagram illustrating an example of a configuration of an optical system of an ophthalmic apparatus according to a second modification example of the embodiments.
FIG. 16 is a schematic diagram illustrating an example of a configuration of an optical system of an ophthalmic apparatus according to a third modification example of the embodiments.
FIG. 17A is a schematic diagram for explaining a conventional ophthalmic apparatus.
FIG. 17B is a schematic diagram for explaining the conventional ophthalmic apparatus.
FIG. 17C is a schematic diagram for explaining the conventional ophthalmic apparatus.

DETAILED DESCRIPTION

The ophthalmic apparatuses disclosed in U.S. Patent No. 7,831,106, U.S. Patent No. 8,237,835, and WO 2021/205965 may cause a center ghost (objective lens surface flare) or a black spot shadow due to a black spot plate arranged in order to suppress an occurrence of generating center ghost, in case of photographing a microcoria eye with a small pupil diameter.

FIG. 17A, FIG. 17B, and FIG. 17C show schematic diagrams for explaining an operation when photographing a fundus of an eye to be examined in a conventional ophthalmic apparatus. FIG. 17A schematically represents an illumination light flux and an imaging light flux in case of photographing the fundus of the eye to be examined. FIG. 17B represents an example of a fundus image in which a center ghost is depicted as an artifact. FIG. 17C represents an example of a fundus image in which a black spot shadow is depicted as an artifact. It should be noted that FIG. 17B and FIG. 17C represent examples of the fundus image formed based on light receiving results obtained by illuminating the fundus with illumination light that has passed through two illumination openings and receiving the imaging light that has passed through a single light receiving opening.

As shown in FIG. 17A, an illumination light flux IL illuminating the fundus of the eye to be examined is transmitted through an objective lens OBJ arranged on an optical axis O, and is guided to the fundus through a pupil of the eye to be examined. An imaging light flux SL, that is a reflection light flux of the illumination light flux IL reflected on the fundus, is emitted from the eye through the pupil, and is transmitted through the objective lens OBJ to guide it to a light receiving optical path (imaging optical path).

The illumination light flux IL and the imaging light flux SL are separated each other at a position substantially conjugate optically to the pupil of the eye to be examined. However, in case that the eye to be examined is a microcoria eye, the illumination light flux IL that enters into the eye through the pupil and the imaging light flux SL come close to each other near an optical axis. As a result, reflected light at a lens surface SF (lens surface near a lens vertex) on the optical axis O of the objective lens OBJ is more likely to enter the light receiving optical path, which may cause center ghost as an artifact. In this case, as shown in FIG. 17B, center ghosts CG1 and CG2 corresponding to the two illumination openings are depicted in the fundus image IMG10 of the eye to be examined. Each of the regions of center ghosts CG1 and CG2 is a region where retinal information is completely lost.

Therefore, in order to prevent the reflected light that causes a center ghost from being guided into the light receiving path, it is common practice in ophthalmic apparatuses to arranged a black spot plate at a position substantially conjugate optically to a vertex of the objective lens OBJ in an illumination light path to block the reflected light from the lens surface SF of the objective lens OBJ. However, in case that the eye to be examined is a high myopia eye, a position conjugate optically to the fundus is shifted, which may cause a black spot shadow due to the black spot plate as an artifact.

In this case, as shown in FIG. 17C, black spot shadows BS1 and BS2 corresponding to the two illumination openings are depicted in the fundus image IMG11 of the eye to be examined. Each of the regions of the black spot shadows BS1 and BS2 becomes a blocked up state (state of blocked up shadows), and is a region where retinal information is completely lost.

In contrast, a method disclosed in Japanese Unexamined Patent Application Publication No. 2020-06172 may enable the adoption of a configuration that does not require the black spot plate. However, in case that the eye to be examined is a microcoria eye, the alignment reference position must be changed, which may causes the control to become more complex.

As described above, in the conventional methods, in case that the eye to be examined is a microcoria eye, either the loss of retinal information on at least some imaging region may occur due to degradation of the fundus image quality caused by the depiction of the center ghost or the black spot shadow, or the control for eliminating the need for the black spot plate may become complicated. These types of problem occur not only when the photographed site is the fundus, but also when photographing the interior of the eye to be examined.

According to embodiments according to the present invention, a new technique for suitably performing small pupil photography while avoiding a loss of intraocular information without complication the control can be provided.

Referring now to the drawings, exemplary embodiments of an ophthalmic apparatus according to the present invention are described below. The contents of the document cited in the present specification can be appropriately incorporated as contents of the following embodiments.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

An ophthalmic apparatus according to embodiments includes an objective lens, an illumination optical system, an optical scanner, a light receiving optical system (imaging optical system, photographing optical system), and a pupil division member. The illumination optical system includes an illumination diaphragm (illumination aperture), and is configured to irradiate slit-shaped illumination light. The illumination diaphragm is arranged at a position substantially conjugate optically to a pupil of an eye to be examined. In the illumination diaphragm, one or more openings (illumination openings, illuminating apertures) are formed at positions eccentric to an optical axis of the illumination optical system. The optical scanner is arranged at a position substantially conjugate optically to the pupil of the eye to be examined, and is configured to deflect the illumination light emitted from the illumination optical system to guide the deflected illumination light to the eye to be examined via the objective lens. The light receiving optical system is configured to guide returning light of the illumination light from the eye to be examined to an image sensor. Here, the returning light has passed through the objective lens. The image sensor is arranged at a position substantially conjugate optically to a photographed site (for example, fundus) of the eye to be examined. The pupil division member includes a photographic diaphragm (imaging diaphragm, imaging aperture) arranged at a position substantially conjugate optically to the pupil of the eye to be examined, and is configured to spatially separate an optical path of the illumination optical system and an optical path of the light receiving optical system. In the photographic diaphragm, one or more openings (light receiving openings, photographing openings (imaging openings)) are formed at positions eccentric to an optical axis of the light receiving optical system.

The returning light from the photographed site is scattered light (reflected light) of the illumination light from the photographed site onto which the illumination light incident on the eye to be examined is irradiated. In some embodiments, the returning light from the photographed site includes scattered light (reflected light) of the illumination light from the photographed site, and fluorescence and its scattered light using the illumination light incident on the photographed site as excitation light.

Shape of each of the one or more openings formed in the illumination diaphragm may be any shape. Shape of each of the one or more openings formed in the photographic diaphragm may be any shape. In some embodiments, the illumination optical system includes the optical scanner. In some embodiments, the light receiving optical system includes the image sensor.

According to such a configuration, the illumination light and the returning light (imaging light) can be spatially separated on the lens surface of the objective lens and inside the lens of objective lens. As a result, it is possible to prevent the reflected light from the lens surface of the objective lens, which causes center ghost (objective lens surface flare), from entering an optical path of the light receiving optical system. Therefore, it becomes no longer necessary to provide a black spot plate, which allows to acquire images of the eye to be examined without blocked up shadows caused to the black spot shadow (without loss of intraocular information such as fundus information). Further, even when photographing a microcoria eye (eye with small pupil), intraocular images, that enable appropriate diagnosis for the microcoria eye, of the eye to be examined can be acquired without complicating the control for photographing the microcoria eye.

In some embodiments, the image sensor is configured to capture a light receiving result in a virtual opening region on a light receiving surface using a rolling shutter method. Here, the virtual opening region corresponds to an irradiated region of the illumination light on the photographed site. The irradiated region is moved in a predetermined scan direction by the optical scanner.

Thereby, even when photographing a microcoria eye, clear intraocular images free from the influence of unwanted scattered light can be acquired without complicating the control for photographing the microcoria eye.

In some embodiments, the one or more openings are formed in the illumination diaphragm and the one or more openings are formed in the photographic diaphragm so that a pupil separation amount becomes greater than a predetermined center ghost occurrence suppression threshold on a pupil conjugate plane at a position substantially conjugate optically to the pupil of the eye to be examined. Here, the pupil separation amount is a shortest distance in an arrangement direction (separation direction) of images of the one or more openings in the illumination diaphragm and images of the one or more opening formed in the photographic diaphragm It should be noted that the arrangement direction is a direction in which the distance between the edges of the images of the one or more openings formed in the illumination diaphragm and the edges of the images of the one or more openings formed in the photographic diaphragm becomes the shortest distance on the pupil conjugate plane.

Thereby, the illumination light flux and the imaging light flux can be reliably separated on the lens surface of the objective lens and inside the lens of objective lens, which allows to reliably suppress the occurrence of center ghost. As a result, the intraocular image suitable for diagnosis can be acquired without the need for a black spot plate.

In some embodiments, the one or more openings are formed in the illumination diaphragm and the one or more openings are formed in the photographic diaphragm so that the pupil separation amount becomes smaller than a predetermined small pupil photographable threshold.

Thereby, even when photographing a microcoria eye, the occurrence of center ghost can be reliably suppressed. As a result, even when photographing a microcoria eye, the intraocular image suitable for diagnosis can be acquired without the need for a black spot plate.

In some embodiments, the ophthalmic apparatus is configured to illuminate a predetermined site of the eye to be examined while moving an irradiated position (illumination region, irradiated range) of slit-shaped illumination light, and to receive returning light from the predetermined site using an image sensor with a one-dimensional or two-dimensional array of light receiving elements. Light receiving result of the returning light is read out from the light receiving element(s) at light receiving position of the returning light corresponding to the irradiated position of the illumination light, in synchronization with the movement timing of the irradiated position of the illumination light. In some embodiments, the predetermined site is an anterior segment or a posterior segment. Examples of the anterior segment include a cornea, an iris, a crystalline lens, a ciliary body, and a ciliary zonule. Examples of the posterior segment include a vitreous body, and a fundus or the vicinity of the fundus (retina, choroid, sclera, etc.).

A method of controlling the ophthalmic apparatus according to the embodiments includes one or more steps executed by a processor (computer) in controlling the ophthalmic apparatus according to the embodiments. A (computers program)/instruction according to the embodiments causes a computer (processor) to execute each step in the method of controlling the ophthalmic apparatus according to the embodiments. A computer program product according to the embodiments includes the computer program(s)/instruction(s). The computer program product realizes each step of the method of controlling the ophthalmic apparatus according to the embodiments, when the computer program(s)/instruction(s) are executed by the processor. A recording medium according to the embodiments is a computer readable non-transitory recording medium (storage medium) on which the program according to the embodiments is recorded (recording). The computer readable recording medium according to the embodiments stores the computer program(s)/instruction(s). The computer readable recording medium realizes each step of the method of controlling the ophthalmic apparatus according to the embodiments, when the computer program(s)/instruction(s) are executed by the processor.

In this specification, the processor includes, for example, a circuit(s) such as, for example, a CPU (central processing unit), a GPU (graphics processing unit), an ASIC (application specific integrated circuit), and a PLD (programmable logic device). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program. At least a part of the storage circuit or the storage apparatus may be included in the processor. Further, at least a part of the storage circuit or the storage apparatus may be provided outside of the processor.

In the following, a case where the photographed site is a fundus will be described. However, the embodiments described below can applied to the case where the photographed site is a site other than the fundus.

Further, in the following, a case where a single opening (aperture) is formed in the illumination diaphragm and a single opening is formed in the photographic diaphragm will be described. However, the embodiments described below can be applied to the case where two or more openings are formed in at least one of the illumination diaphragm or the photographic diaphragm.

[Configuration of optical system]

FIG. 1, FIG. 2, and FIG. 3 show schematic diagrams of a configuration of an optical system of the ophthalmic apparatus according to the embodiments. FIG. 1 represents an example of a configuration of the optical system of the ophthalmic apparatus according to the embodiments. In FIG. 1, a fundus conjugate position P, that is a position conjugate optically to a fundus Ef of the eye E to be examined, and a pupil (iris) conjugate position Q, that is a position substantially conjugate optically to a pupil (iris) Eu of the eye E to be examined, are illustrated. FIG. 2 schematically represents an example of a configuration of an iris aperture 21 in FIG. 1 when viewed from a direction of an optical axis O. FIG. 3 schematically represents a pupil division member 45 in FIG. 1, by corresponding a front view when viewing from the direction of the optical axis O with a side view when viewing form a direction perpendicular to the optical axis O. In FIG. 1, FIG. 2, and FIG. 3, like parts are designated by like reference numerals as in repetitious description of such parts may not be provided.

An ophthalmic apparatus 1 according to the embodiments includes a light source 10, an illumination optical system 20, an optical scanner 30, a projection optical system 35, an imaging optical system 40, and an imaging device 50. In some embodiments, the illumination optical system 20 includes at least one of the light source 10, the optical scanner 30, or the projection optical system 35. In some embodiments, the imaging optical system 40 includes the imaging device 50. In some embodiments, the projection optical system 40 includes the optical scanner 30.

(Light source 10)

The light source 10 includes a visible light source that generates light in the visible region. For example, the light source 10 generates light having a central wavelength in the wavelength range of 420 nm to 700 nm. This type of light source 10 includes, for example, an LED (Light Emitting Diode), an LD (Laser Diode), a halogen lamp, or a xenon lamp. In some embodiments, the light source 10 includes a white light source or a light source capable of outputting light with each color component of RGB. In some embodiments, the light source 10 includes a light source capable of switching to output the light in infrared region or the light in visible region. For example, the light source 10 is arranged at a position non-conjugate optically to the fundus Ef and the iris, respectively.

(Illumination optical system 20)

The illumination optical system 20 generates slit-shaped illumination light using the light from the light source 10. The illumination optical system 20 guides the generated illumination light to the optical scanner 30.

The illumination optical system 20 includes the iris aperture 21, a slit 22, and a relay lens 23. The light from the light source 10 passes through openings (one or more openings) formed in the iris aperture 21, passes through opening(s) formed in the slit 22, and is transmitted through the relay lens 23. The relay lens 23 includes one or more lenses. The light transmitted through the relay lens 23 is guided to the optical scanner 30.

(Iris aperture 21)

The iris aperture 21 (specifically, opening(s) described below) can be disposed at a position substantially conjugate optically to the iris (pupil) Eu of the eye E to be examined ( (pupil (iris) conjugate position Q or in the vicinity of the pupil (iris) conjugate position Q)), as an illumination diaphragm. In the iris aperture 21, one or more openings (illumination openings) are formed at position(s) eccentric to the optical axis O. In the present embodiment, in the iris aperture 21, a single opening is formed at a position eccentric to the optical axis O.

For example, as shown in FIG. 2, in the iris aperture 21, a square-shaped opening 21A is formed at a position away from the optical axis O of the illumination optical system 20. A shape of the opening 21A may be rectangular or parallelogram. Alternatively, the shape of the opening 21A may be triangular, a polygon with five or more sides, elliptical, or circular. The opening formed in the iris aperture 21 defines an incident position (incident shape) of the illumination light on the iris (pupil) Eu of the eye E to be examined. For example, by forming the opening 21A as shown in FIG. 2, when the pupil center of the eye E to be examined is arranged on the optical axis O, the illumination light can enter into the eye from positions eccentric to the pupil center.

Further, light quantity distribution of the light passing through the opening formed in the iris aperture 21 can be changed by changing a relative position between the light source 10 and the opening formed in the iris aperture 21. For example, the relative position between the light source 10 and the opening(s) formed in the iris aperture 21 can be changed based on a dioptric power (refractive power) of the eye E to be examined or image quality evaluation information on at least a portion of the fundus image obtained by the ophthalmic apparatus 1.

(Slit 22)

The slit 22 (specifically, its opening(s) (its aperture(s))) can be disposed at a position substantially conjugate optically to the fundus Ef of the eye E to be examined (fundus conjugate position P or in the vicinity of the fundus conjugate position P). In the slit 22, a single slit-shaped (line-shaped) opening (slit opening) is formed. For example, in the slit 22, the opening is formed extending in a direction corresponding to a line direction (row direction) that is read out from the image sensor 51 described below using the rolling shutter method. For example, in the slit 22, the opening is formed so that a direction perpendicular to the movement direction of light receivable range (virtual opening range) of the returning light on the light receiving surface of the image sensor 51 becomes a slit direction (longitudinal direction of the slit). The opening formed in the slit 22 defines an irradiated pattern of the illumination light on the fundus Ef of the eye E to be examined.
The slit 22 can be moved in the optical axis direction of the illumination optical system 20 using a movement mechanism (movement mechanism 22D described below). The movement mechanism moves the slit 22 in the optical axis direction, under the control from a controller 100 described below. For example, the controller 100 controls the movement mechanism in accordance with a state of the eye E to be examined. This allows to move the position of the slit 22 in accordance with the state (specifically, the dioptric power or the shape of the fundus Ef) of the eye E to be examined.
In some embodiments, the slit 22 is configured so that at least one of the position of the opening or the shape of the opening can be changed in accordance with the state of the eye E to be examined without being moved in the optical axis direction. The function of the slit 22 with these functions is, for example, realized by a liquid crystal shutter.
The light from the light source 10 that has passed through the opening formed in the iris aperture 21 is output as the slit-shaped illumination light by passing through the opening formed in the slit 22. The slit-shaped illumination light is transmitted through the relay lens 23, and is guided to the optical scanner 30.

(Optical scanner 30)

The optical scanner 30 (specifically, deflection surface) is disposed at a position conjugate optically to the pupil (iris) Eu of the eye E to be examined (pupil conjugate position Q or in the vicinity of the pupil conjugate position Q). The optical scanner 30 deflects the slit-shaped illumination light transmitted through the relay lens 23 (slit-shaped light passing through the opening formed in the slit 22). Specifically, the optical scanner 30 deflects the slit-shaped illumination light to guide the deflected illumination light to the projection optical system 35, while changing a deflection angle within a predetermined deflection angular range with the pupil (iris) Eu of the eye E to be examined or in the vicinity of the pupil (iris) of the eye E to be examined as a scan center position. Thereby, the slit-shaped illumination light is sequentially irradiated onto a predetermined illumination region on the fundus Ef
The optical scanner 30 can deflect the illumination light one-dimensionally or two-dimensionally. In case that the optical scanner 30 deflects the illumination light one-dimensionally, the optical scanner 30 includes a galvano scanner that deflects the illumination light within a predetermined deflection angular range with reference to a predetermined deflection reference direction. In case that the optical scanner 30 deflects the illumination light two-dimensionally, the optical scanner 30 includes a first galvano scanner and a second galvano scanner. The first galvano scanner deflects the illumination light so as to move the irradiated position of the illumination light in a horizontal direction orthogonal to the optical axis of the illumination optical system 20. The second galvano scanner deflects the illumination light deflected by the first galvano scanner so as to move the irradiated position of the illumination light in a vertical direction orthogonal to the optical axis of the illumination optical system 20. Examples of scan mode for moving the irradiated position of the illumination light using the optical scanner 30 include a horizontal scan, a vertical scan, a cross scan, a radial scan, a circle scan, a concentric scan, and a helical (spiral) scan.

(Projection optical system 35)

The projection optical system 35 guides the illumination light deflected by the optical scanner 30 to the fundus Ef of the eye E to be examined. In the present embodiment, the projection optical system 35 is configured to guide the illumination light deflected by the optical scanner 30 to the fundus Ef through an optical path coupled with an optical path of the imaging optical system 40 by the pupil division member 45 as an optical path coupling member described below.
The projection optical system 35 includes a relay lens 41, a reflective mirror 42, and a relay lens 43. Each of the relay lenses 41 and 43 includes one or more lenses.
In the projection optical system 35, the illumination light deflected by the optical scanner 30 is transmitted through the relay lens 41, is reflected by the reflective mirror 42, is transmitted through the relay lens 43, and is guided to the pupil division member 45.
In some embodiments, the illumination optical system 20 includes the optical scanner 30 and the projection optical system 35.

(Imaging optical system 40)

The imaging optical system 40 guides the illumination light that has been guided through the projection optical system 35 to the fundus Ef of the eye E to be examined, and also guides the returning light of the illumination light from the fundus Ef to the imaging device 50.

In the imaging optical system 40, an optical path of the illumination light from the projection optical system 35 and an optical path of the returning light of the illumination light from the fundus Ef are coupled. By using the pupil division member 45 as the optical path coupling member for coupling these optical paths, the optical path of the illumination light and the optical path of the returning light of the illumination light can be spatially divided (pupil-divided) on the pupil conjugate plane at the position substantially conjugate optically to the pupil Eu of the eye E to be examined.

The imaging optical system 40 includes the pupil division member 45, the objective lens 46, a focusing lens 47, a relay lens 48, and an imaging lens 49. The relay lens 48 includes one or more lenses.

(Pupil division member 45)

The pupil division member 45 includes a photographic diaphragm, and is configured to spatially separate the optical path of the illumination optical system 20 (optical path of the illumination light from the projection optical system 35) and the optical path of the imaging optical system 40. In the present embodiment, the pupil division member 45 is a reflective mirror with photographic diaphragm. Here, the photographic diaphragm is arranged at a position substantially conjugate optically to the pupil Eu of the eye E to be examined (pupil conjugate position Q or in the vicinity of the pupil conjugate position Q). In the photographic diaphragm, one or more openings (light receiving openings, photographing openings (imaging openings)) are formed at positions eccentric to an optical axis of the imaging optical system 40. In the present embodiment, a single opening is formed in the photographic diaphragm. Further, the pupil division member 45 includes a reflective mirror configured to deflect the illumination light deflected by the optical scanner 30 toward the eye to be examined (specifically, objective lens 46).

The pupil division member 45 approximately coaxially couples the optical path of the illumination light from the projection optical system 35 (illumination optical system 20) with the optical system of the imaging optical system 40. Therefore, the optical axis of the common optical path of the illumination light and the returning light in the imaging optical system 40 substantially coincides with the optical axis O of the illumination optical system 20.

Alternatively, the pupil division member 45 includes a base substance 45a and a reflection member 45b, as shown in FIG. 3, for example. In this case, one or more openings (light receiving openings, photographing openings) are formed in the base substance 45a, for example. In the configuration shown in FIG. 3, the one or more openings formed in the base substance 45a function as the photographic diaphragm. In the present embodiment, a single opening 45A is formed in the base substance 45a. The reflection member 45b deflects the illumination light from the projection optical system 35 toward the objective lens 46.

In some embodiments, the base substance 45a includes the photographic diaphragm, in which a single opening 45A is formed.

In FIG. 3, the reflection member 45b is provided on a surface of the base substance 45a. The base substance 45a and the reflection member 45b are provided with the opening 45A at a position eccentric to the optical axis O of the imaging optical system 40. For example, the pupil division member 45 may be a hole mirror in which the reflection member 45b is provided around the opening 45A formed as the photographic diaphragm. For example, the reflection member 45b may be provided around the opening 45A formed in the photographic diaphragm provided on the base substance 45a.

In some embodiments, the reflection member 45b is formed by evaporating a reflective film onto a surface of the base substance 45a. The reflective film may be a metal film or a dielectric multi-layer film. The reflective film is formed by evaporating (mirror-depositing) a metal film onto the surface of base substance 45a, or by evaporating the dielectric substance on multiple layers on the surface of base substance 45a.

In some embodiments, the base substance 45a may be a transparent member. In this case, it is sufficient for the opening 45A to be formed in the reflection member 45b provided on the surface of the base substance 45a.

When the position matching of the optical system relative to the eye E to be examined is completed, a position corresponding to the optical axis O in the pupil division member 45 is arranged at a position substantially conjugate optically to the pupil Eu of the eye E to be examined. In this case, the illumination light from the projection optical system 35 is reflected by the reflection member 45b, and is guided to the objective lens 46. The returning light of the illumination light from the eye E to be examined passes through the opening 45A, and is guided to the imaging optical system 40. The pupil division member 45 according to the embodiments is configured to spatially divide (separate) the illumination light and the returning light from the eye to be examined on the pupil conjugate plane at the position substantially conjugate optically to the pupil of the eye E to be examined.

FIG. 4 shows an explanatory diagram of the pupil conjugate plane according to the embodiments. FIG. 4 schematically represents an image IPI of the illumination opening and an image SPI of the light receiving opening on the pupil conjugate plane PL. In FIG. 4, like reference numerals designate like parts as in FIGs. 1 to 3. The same description may not be repeated below.

By performing pupil division with the pupil division member 45 described above, the image IPI of the illumination opening, that is an image of the opening formed in the iris aperture 21, and the image SPI of the light receiving opening, that is an image of the opening formed in the photographic diaphragm (pupil division member 45) are spatially divided on the pupil conjugate plane PL. In this case, the image IPI of the illumination opening and the image SPI of the light receiving opening are divided by a pupil separation amount GP. Here, the pupil separation amount GP corresponds to the shortest distance in the arrangement direction (separation direction) between the image IPI of the illumination opening and the image SPI of the light receiving opening.

By increasing the pupil separation amount GP, the overlap of the light flux cross-sections between the illumination light and the returning light on the lens surface of the objective lens 46 or inside the objective lens 46 can be reliably avoided. As a result, the occurrence of center ghost can be suppressed without requiring a black spot plate. Therefore, it is desirable that the opening 21A (or one or more openings) is formed in the iris aperture 21 and the opening 45A (or one or more openings) is formed in the pupil division member 45 (photographic diaphragm) so that the pupil separation amount GP becomes greater than a predetermined center ghost occurrence suppression threshold.

In this case, as shown in FIG. 3, it is desirable that the opening 21A (or one or more openings) is formed in the iris aperture 21 and the opening 45A (or one or more openings) is formed in the pupil division member 45 (photographic diaphragm) so that the pupil separation amount GP is approximately constant in a direction perpendicular to the arrangement direction. Here, the arrangement direction is an arrangement direction of the image(s) of the opening 21A (or one or more openings) formed in the iris aperture 21 and the images(s) of the opening 45A (or one or more openings) formed in the pupil division member 45 on the pupil conjugate plane. Thereby, it becomes possible to maximize the pupil separation amount GP while ensuring light quantity by increasing the sizes of the openings 21A and 45A.

On the other hand, when the pupil separation amount GP is increased, the size of the pupil diameter that can be imaged becomes larger, which makes it impossible to image (photograph) a microcoria eyes. Therefore, it is desirable that the opening 21A (or one or more openings) is formed in the iris aperture 21 and the opening 45A (or one or more openings) is formed in the pupil division member 45 (photographic diaphragm) so that the pupil separation amount GP becomes smaller than a predetermined small pupil photographable threshold.

The center ghost occurrence suppression threshold and the small pupil photographable threshold described above will be described below.

(Focusing lens 47)

The focusing lens 47 can be moved in an optical axis direction of the imaging optical system 40 using a movement mechanism (not shown). The movement mechanism moves the focusing lens 47 in the optical axis direction under the control from the controller 100 described below. This allows to image the returning light of the illumination light having passed through the opening 45A formed in the pupil division member 45 on the light receiving surface of the image sensor 51 in the imaging device 50 in accordance with the state of the eye E to be examined.

In such an imaging optical system 40, the illumination light from the projection optical system 35 is reflected toward the objective lens 46 on the reflection member 45b. The illumination light reflected on the reflection member 45b is refracted by the objective lens 46, enters into the eye through the pupil of the eye E to be examined, and illuminates the fundus Ef of the eye E to be examined.

The returning light of the illumination light from the fundus Ef is refracted by the objective lens 46, passes through the opening 45A formed in the pupil division member 45, is transmitted through the focusing lens 47, is transmitted through the relay lens 48, and is imaged on the light receiving surface of the image sensor 51 in the imaging device 50 through the imaging lens 49.

(Imaging device 50)

The imaging device 50 includes the image sensor 51 that receives the returning light of the illumination light that has been guided from the fundus Ef of the eye E to be examined through the imaging optical system 40. The imaging device 50 can output the light receiving result of the returning light under the control from the controller 100 described below.

(Image sensor 51)

The image sensor 51 realizes the function as a pixelated photodetector. The light receiving surface (detecting surface, imaging surface) of the image sensor 51 can be arranged at a position substantially conjugate optically to the fundus Ef.

The light receiving result acquired by the image sensor 51 is read out using a rolling shutter method. In some embodiments, the controller 100 described below performs readout control of the light receiving result by controlling the image sensor 51. In some embodiments, the image sensor 51 can automatically output the light receiving result(s) for a predetermined number of lines, along with information indicating the light receiving position(s).

Such an image sensor 51 is a complementary metal-oxide-semiconductor (CMOS) image sensor. In this case, the image sensor 51 includes a plurality of pixels arranged two-dimensionally, a plurality of vertical signal lines, and a horizontal signal line. Each pixel includes a photodiode (light receiving element), and a capacitor. The vertical signal lines are provided for each pixel group in the column direction (vertical direction) orthogonal to the row direction (horizontal direction). Each of the vertical signal lines is selectively electrically connected to the pixel group in which the electrical charge corresponding to the light receiving result is accumulated. The horizontal signal line is selectively electrically connected to the vertical signal lines. Each of the pixels accumulates the electrical charge corresponding to the light receiving result of the returning light. The accumulated electrical charge is read out sequentially for each pixel group in the row direction, for example. For example, for each line in the row direction, a voltage corresponding to the electrical charge accumulated in each pixel is supplied to the vertical signal line. The vertical signal lines are selectively electrically connected to the horizontal signal line. By performing readout operation for each line in the row direction described above sequentially in the vertical direction, the light receiving results of the plurality of pixels arranged two-dimensionally can be read out.

By capturing (reading out) the light receiving results of the returning light using the rolling shutter method for this type of image sensor 51, the light receiving image corresponding to a desired virtual opening (aperture) shape extending in the row direction is acquired. Such control is disclosed in, for example, U.S. Patent No. 8,237,835.

FIG. 5 shows an explanatory diagram of an operation of the ophthalmic apparatus 1 according to the embodiments. FIG. 5 schematically represents an irradiated range IP of the slit-shaped illumination light irradiated on the fundus Ef and a virtual opening range (light receivable range) OP on the light receiving surface SR of the image sensor 51.

For example, the controller 100 described below deflects the slit-shaped illumination light formed by the illumination optical system 20, using the optical scanner 30. Thereby, the irradiated range IP of the slit-shaped illumination light is sequentially moved in a slit direction (for example, the vertical direction) orthogonal to the slit direction (for example, the row direction, the horizontal direction) on the fundus Ef.

On the light receiving surface SR of the image sensor 51, for example, by changing the pixels to be captured in units of lines by the controller 100 described below, the virtual opening range OP is set. The opening range OP is preferable to be the light receiving range IP' of the returning light of the illumination light on the light receiving surface SR or wider than the light receiving range IP'. For example, the controller 100 described below performs the movement control of the opening range OP in synchronization with the movement control of the irradiated range IP of the illumination light. Thereby, without being affected by unnecessary scattered light, high quality images of the fundus Ef with strong contrast can be acquired using a simple configuration.

FIG. 6 and FIG. 7 schematically show examples of a control timing of the rolling shutter method for the image sensor 51. FIG. 6 represents an example of a timing of the readout control for the image sensor 51. FIG. 7 represents a timing of the movement control for the irradiated range IP (the light receiving range IP') superimposed on the timing of the readout control in FIG. 6. In FIG. 6 and FIG. 7, the horizontal axis represents the number of rows in the image sensor 51, and the vertical axis represents time.

In addition, in FIG. 6 and FIG. 7, for convenience of explanation, it is assumed that the number of rows in the image sensor 51 is 1920. However, the configuration according to the embodiments is not limited to the number of rows. Further, in FIG. 7, for convenience of explanation, it is assumed that the slit width (width in the row direction) of the slit-shaped illumination light is 40 rows.

The readout control in the row direction includes a reset control, an exposure control, a charge transfer control, and an output control. The reset control is a control that initializes an amount of electrical charge accumulated in the pixels in the row direction. The exposure control is a control that illuminates light on the photodiode and accumulates the electrical charge corresponding to the amount of received light in the capacitor. The charge transfer control is a control that transfers the amount of the electrical charge accumulated in the pixel to the vertical signal line. The output control is a control that outputs the amount of the electrical charge accumulated in the plurality of vertical signal lines via the horizontal signal line. That is, as shown in FIG. 6, the readout time T for reading out the electrical charge accumulated in the pixels in the row direction is the sum of the time Tr required for the reset control, the time Te (exposure time) required for the exposure control, the time Tc required for the charge transfer control, and the time Tout required for the output control.

In FIG. 6, by shifting the readout (capturing) start timing (start timing of time Tc) in units of rows, the light receiving results (amount of electrical charge) accumulated in the pixels in the desired range in the image sensor 51 are acquired. For example, in case that the pixel range shown in FIG. 6 is for a single frame of the image, the frame rate FR is determined uniquely.

In the present embodiment, the irradiated position of the illumination light on the fundus Ef, the illumination light having a slit width for a plurality of rows, is sequentially shifted in a direction corresponding to the column direction on the fundus Ef.

For example, as shown in FIG. 7, at each predetermined shift time $\Delta t$, the irradiated position of the illumination light on the fundus Ef is shifted in row units in the direction corresponding to the column direction. The shift time $\Delta t$ is obtained by dividing the exposure time Te of the pixel in the image sensor 51 by the slit width (e.g., the number of rows of the slit width = 40) of the illumination light ($\Delta t$ = Te / 40). Synchronized with this movement timing of this irradiated position, the readout start timing of each row of pixels is delayed and is started for each row in units of shift time $\Delta t$. This allows to acquired high quality images of the fundus Ef with strong contrast in a short time with a simple control.

In some embodiments, the image sensor 51 is configured using one or more line sensors.

In the embodiments, as described above, by setting the pupil separation amount GP to be greater than the center ghost occurrence suppression threshold and smaller than the small pupil photographable threshold, small pupil photography can be performed while suppressing the occurrence of the center ghost without providing a black spot plate.

Hereinafter, the center ghost occurrence suppression threshold and the small pupil photographable threshold according to the embodiments will be described.

The center ghost occurrence suppression threshold is obtained by identifying a condition where an intersection point of the illumination beam and the light receiving beam (imaging beam) is not positioned on the lens surface of the objective lens 46 or inside the lens of the objective lens 46. Such a center ghost occurrence suppression threshold can be determined based on a working distance of the ophthalmic apparatus 1, a dioptric power of an eye E to be examined that can be imaged, a viewing angle of the illumination light with respect to the fundus Ef (photographed site), and a viewing angle of the returning light with respect to the fundus Ef. Here, by setting the dioptric power to a dioptric power of the high myopia eye, even if the eye E to be examined is a high myopia eye, the occurrence of the center ghost can be suppressed without providing a black spot plate.

The small pupil photographable threshold can be determined based on a predetermined pupil diameter for small pupil photography, the pupil separation amount GP on the pupil conjugate plane PL, a width of the opening 21A (or one or more openings) in the arrangement direction, and a width of the opening 45A (or one or more openings) in the arrangement direction. Here, the arrangement direction is an arrangement direction of the image(s) of the opening 21A (or one or more openings) formed in the iris aperture 21 and the images(s) of the opening 45A (or one or more openings) formed in the pupil division member 45 on the pupil conjugate plane.

[Center ghost occurrence suppression threshold]

FIG. 8 schematically shows the illumination beam and the light receiving beam according to the embodiments. FIG. 8 schematically represents a two-dimensional x-y coordinate system for expressing the intersection point of the illumination beam and the light receiving beam with x-position and y-position. Hereinafter, all distances are assumed to be path lengths.

In the x-y coordinate system shown in FIG. 8, it is assumed that a direction of the optical axis O of the optical system of the ophthalmic apparatus 1 from a pupil center C toward the fundus Ef is the x-direction and the arrangement direction, which is perpendicular to the x-direction, of the image of the opening 21A and image of the opening 45A at a pupil position is the y-direction.

Hereinafter, it is assumed that a focal point distance of an crystalline lens of the eye E to be examined is "f", an origin position is the pupil center C of the eye E to be examined, a distance in the x-direction from the pupil center C toward the fundus Ef is "$L_f$" (>0), and a distance in the x-direction from the pupil center C toward the fundus conjugate position arranged in a direction toward the apparatus side is "$L_{f'}$" (>0). Further, it is assumed that a distance in the x-direction from the pupil center C to the intersection point of the illumination beam $W_{IL}$ and the light receiving beam $W_{SL}$ is "$L_0$".

When the intersection point position of the illumination beam $W_{IL}$ and the light receiving beam $W_{SL}$ exists on the lens surface of the objective lens 46 or inside the lens of the objective lens 46, the center ghost (objective lens surface flare) occurs. Therefore, when the intersection point position described above exists outside the lens of the objective lens 46, the occurrence of the center ghost can be suppressed.

It is assumed that, in obtaining the intersection point position, the illumination beam passes through the lower edge of the image of the illumination opening (opening 21A formed in the iris aperture 21) at the pupil Eu and the lower edge of the opening (slit opening) formed in the slit 22 at the fundus conjugate position.

Here, it is assumed that a distance from the optical axis to the lower edge of the image of the illumination opening is "G", a width of the opening formed in the slit 22 is "$d_G$", and an optical magnification from the fundus to the fundus conjugate position is "$\beta$". The illumination beam described above passes a position (0, G) of the lower edge of the image of the illumination opening and a position ($L_f'$, $-\beta \times d_G / 2$) of the lower edge of the slit opening at the fundus conjugate position. Thus, the relationship can be expressed as shown in formula (1).

[Equation 1]

$$y = \frac{1}{L_f{}'} \times \left(\frac{1}{2} \times \beta \times d_G + G\right) \times x + G \quad (1)$$

In the same way, it is assumed that, in obtaining the intersection point position, the light receiving beam passes through the upper edge of the image of the light receiving opening at the pupil Eu and the upper edge of an exposure width on the light receiving surface of the image sensor 51 at the fundus conjugate position.

Here, it is assumed that a distance from the optical axis to the upper edge of the light receiving opening is "H", and the exposure width on the light receiving surface of the image sensor 51 at the fundus conjugate position is "dH". The light receiving beam described above passes a position (0, - H) of the upper edge of the image of the light receiving opening and a position (-Lf', β × d H / 2) of the upper edge of the exposure width on the light receiving surface of the image sensor 51 at the fundus conjugate position. Thus, the relationship can be expressed as shown in formula (2).

[Equation 2]

$$y = -\frac{1}{L_f{}'} \times \left(\frac{1}{2} \times \beta \times d_H + H\right) \times x - H \quad (2)$$

The x-coordinate of the intersection point between the illumination beam and the light receiving beam described above can be expressed as shown in formula (3) based on formula (1) and formula (2).

[Equation 3]

$$x = -\frac{2 \times L_f' \times (G + H)}{2 \times (G + H) + \beta \times (d_G + d_H)} \quad (3)$$

Thus, the distance L0 can be expressed as shown in formula (4) based on formula (3).

[Equation 4]

$$L_0 = |x| = \left| -\frac{2 \times L_f' \times (G + H)}{2 \times (G + H) + \beta \times (d_G + d_H)} \right| \quad (4)$$

In case that the distance L0 between the intersection point position described above and the pupil center C equals to the working distance WD of the ophthalmic apparatus 1, the pupil separation amount (G0 + H0) can be obtained when the intersection point position of the illumination beam and the light receiving beam coincides with the lens surface of the objective lens 46 (see formula (5)).

[Equation 5]

$$L_0 = |x| = \left| -\frac{2 \times L_f' \times (G_0 + H_0)}{2 \times (G_0 + H_0) + \beta \times (d_G + d_H)} \right| = \frac{2 \times L_f' \times (G_0 + H_0)}{2 \times (G_0 + H_0) + \beta \times (d_G + d_H)} = WD \quad (5)$$

Here, it is assumed that the dioptric power of the eye E to be examined is "D". In case that the eye E to be examined is a high myopia eye, "D" becomes a negative value. Further, formula (6) is satisfied between "Lf" and "D".

[Equation 6]

$$-L_f{}' = \frac{1000}{D} \quad (6)$$

Furthermore, formula (7) is satisfied among "Lf", "D", and "f' from and "D" from paraxial imaging formula.

[Equation 7]

$$L_f = \frac{1000 \times f}{1000 + D \times f} \quad (7)$$

Here, the optical magnification "β" described above can be expressed as shown in formula (8).

[Equation 8]

$$\beta = \frac{L_f'}{L_f} = -\frac{D \times f + 1000}{D \times f} \qquad (8)$$

By substituting formulas (6) to (8) into "β" and "Lf" in formula (5), the pupil separation amount (Go + Ho) in formula (5) can be expressed as shown in formula (9).
[Equation 9]

$$(G_0 + H_0) = \frac{WD \times (D \times f + 1000) \times (d_G + d_H)}{2 \times f \times (1000 + D \times WD)} \qquad (9)$$

Here, "dG" and "dH" in FIG. 8 can be expressed using a viewing angle with respect to the fundus Ef as the photographed site.

FIG. 9 shows an explanatory diagram of the viewing angle according to the embodiments. In FIG. 9, the same parts as in FIG. 8 are shown by the same symbols and their descriptions will be omitted in an appropriate manner.

As shown in FIG. 9, when the viewing angle of the illumination light with respect to the fundus Ef is "α" in the x-y coordinate system same as FIG. 8, "dG" in FIG. 8 can be expressed using the viewing angle "α" of the illumination light with respect to the fundus Ef as shown in formula (10). In the same way, when the viewing angle of the returning light with respect to the fundus Ef at the fundus conjugate position (fundus position) is "γ" in the x-y coordinate system, "d H" in FIG. 8 can be expressed using the viewing angle of the returning light with respect to the fundus Ef as shown in formula (11).

[Equation 10]

$$d_G = 2 \times L_f \times \tan\left(\frac{\alpha}{2}\right) = 2 \times \frac{1000 \times f}{1000 + D \times f} \times \tan\left(\frac{\alpha}{2}\right) \qquad (10)$$

$$d_H = 2 \times L_f \times \tan\left(\frac{\gamma}{2}\right) = 2 \times \frac{1000 \times f}{1000 + D \times f} \times \tan\left(\frac{\gamma}{2}\right) \qquad (11)$$

Thus, formula (9) becomes formula (12) by substituting formulas (10) and (11) into formula (9).
[Equation 11]

$$(G_0 + H_0) = \frac{1000 \times WD}{1000 + D \times WD} \times \left\{\tan\left(\frac{\alpha}{2}\right) + \tan\left(\frac{\gamma}{2}\right)\right\} \qquad (12)$$

It is clear from FIG. 8 that increasing the pupil separation amount GP (= G + H) shifts the intersection point position described above toward the apparatus side. Therefore, in order to suppress the occurrence of the center ghost (objective lens surface flare), it is sufficient for the intersection point position to be located on the apparatus side relative to the lens surface of the objective lens 46. The pupil separation amount GP (= G + H) in this case just needs to be greater than the pupil separation amount (Go + Ho) when the intersection point position coincides with the lens surface of the objective lens 46. Thus, formula (13) needs just to be satisfied. In other words, the pupil separation amount (Go + Ho) is the center ghost occurrence suppression threshold.
[Equation 12]

$$GP = (G + H) > (G_0 + H_0) = \frac{1000 \times WD}{1000 + D \times WD} \times \left\{\tan\left(\frac{\alpha}{2}\right) + \tan\left(\frac{\gamma}{2}\right)\right\} \qquad (13)$$

[Small pupil photographable threshold]

Further, in order to enable small pupil photography, the following relationship needs just to be satisfied between the pupil separation amount (G + H) and the pupil diameter φ for small pupil photography.

FIG. 10 schematically shows an image P1 of the illumination opening and an image P2 of the light receiving opening on the pupil conjugate plane according to the embodiments. In FIG. 10, the same parts as in FIG. 4 are shown by the same symbols and their descriptions will be omitted in an appropriate manner.

In order to perform small pupil photography, a sum of the pupil separation amount (G + H), a width "g" in the arrangement direction of the image P1 of the illumination opening, and a width "h" in the arrangement direction of the image P2 of the light receiving opening, in the arrangement direction of the image P1 of the illumination opening and the image P2 of the light receiving opening, needs to be smaller than the pupil diameter φ for small pupil photography (formula (15)).

Here, the width "g" corresponds to a width of the opening 21A (or one or more openings) in the arrangement direction described above. The width "h" corresponds to a width of the opening 45A (or one or more openings) in the arrangement direction described above. In other words, (φ - (g + h)) is the small pupil photographable threshold.

As described above, by setting the pupil separation amount so as to satisfy formulas (14) and (15), even if the eye E to be examined is a high myopia eye, the small pupil photography can be performed while suppressing the occurrence of the center ghost without providing a black spot plate.

[Equation 13]

$$(G+H) > \frac{1000 \times WD}{1000 + D \times WD} \times \left\{\tan\left(\frac{\alpha}{2}\right) + \tan\left(\frac{\gamma}{2}\right)\right\} \qquad (14)$$

$$\emptyset > (G+H) + g + h \qquad (15)$$

[Configuration of processing system]

As shown in FIG. 11, the processing system (control system) of the ophthalmic apparatus 1 is configured with the controller 100 as a center. It should be noted that at least a part of the configuration of the processing system may be included in the ophthalmic apparatus 1.

(Controller 100)

The controller 100 controls each part of the ophthalmic apparatus 1. The controller 100 includes a main controller 101 and a storage unit 102. The main controller 101 includes a processor and executes the control processing of each part of the ophthalmic apparatus 1 by executing processing according to the program(s) stored in the storage unit 102.

(Main controller 101)

The main controller 101 performs control for the light source 10, control for a movement mechanism 10D, control for the illumination optical system 20, control for the optical scanner 30, control for the imaging optical system 40, control for the imaging device 50, and control for the image forming unit 200. Further, the main controller 101 can control an operation unit 110 and a display unit 120. The main controller 101 can control each part of the ophthalmic apparatus 1 based on operation information input via the operation unit 110.

The control for the light source 10 includes switching the light source on and off (or switching the wavelength region of the light), and changing the light quantity of the light source.

The movement mechanism 10D changes at least one of the position of the light source 10 or the orientation of the light source 10 using a known mechanism. The main controller 101 can change at least one of a relative position of the light source 10 to the iris aperture 21 and the slit 22, or a relative orientation of the light source 10 to the iris aperture 21 and the slit 22.

The control for the illumination optical system 20 includes control for a movement mechanism 22D. The movement mechanism 22D moves the slit 22 in the optical axis direction of the illumination optical system 20. The main controller 101 controls the movement mechanism 22D in accordance with the state of the eye E to be examined to arrange the slit 22 at the position corresponding to the state of the eye E to be examined. Examples of the state of the eye E to be examined includes a shape of the fundus Ef, a dioptric power (refractive power), and an axial length. The dioptric power can be acquired from a known eye refractive power measurement apparatus as disclosed in Japanese Unexamined Patent Application No. 61- 293430 or Japanese Unexamined Patent Application Publication No. 2010-259495, for example. The axial length can be obtained from a known axial length measurement apparatus or a measurement value acquired by an optical coherence tomography.

For example, the storage unit 102 stores first control information. In the first control information, the positions of the slit 22 on the optical axis of the illumination optical system 20 are associated with the dioptric powers in advance. The main controller 101 identifies the position of the slit 22 corresponding to the dioptric power by referring to the first control information, and controls the movement mechanism 22D so as to arrange the slit 22 at the identified position.

Here, as the slit 22 moves, the light quantity distribution of the light passing through the aperture formed in the slit 22 changes. In this case, as described above, the main controller 101 can control the movement mechanism 10D to change at least one of the position of the light source 10 or the orientation of the light source 10.

The control for the optical scanner 30 includes set a deflection start angle and a deflection end angle of the deflection surface for deflecting the illumination light, and control of the angle of the deflection surface. By controlling an angular range of the deflection surface, the scan range (scan start position and scan end position) can be controlled. By controlling the speed of changing the angle of deflection surface, the scan speed can be controlled.

The control for the imaging optical system 40 includes control for a movement mechanism 47D. The movement mechanism 47D moves the focusing lens 47 in the optical axis direction of the imaging optical system 40. The main controller 101 can control the movement mechanism 47D based on an analysis result of the image acquired using the image sensor 51. Further, the main controller 101 can control the movement mechanism 47D based on an operation content of the user using the operation unit 110.

The control for the imaging device 50 includes a control for the image sensor 51. The control for the image sensor 51 includes a control for reading out the light receiving result using a rolling shutter method (for example, setting of light receiving size corresponding to the size of the illumination pattern, or the like). Further, the control for the image sensor 51 includes the reset control, the exposure control, the charge transfer control, and the output control. The time Tr required for the reset control, the time (exposure time) Te required for the exposure control, the time Tc required for the charge transfer control, and the time Tout required for the output control, etc., can be changed.

Examples of the control for the image forming unit 200 include various kinds of image processing and various kinds of analysis processing on the light receiving results acquired from the image sensor 51, and image forming processing using the light receiving results. Examples of the image processing include noise removal processing on the light receiving results, and luminance (brightness) correction processing for easily identifying a predetermined site depicted in the light receiving image based on the light receiving results. Examples of the analysis processing include an identifying processing of the in-focus state.

The image forming unit 200 can form the light receiving image (image) corresponding to the arbitrary opening range based on the light receiving result(s) read out from the image sensor 51 using the rolling shutter method. The image forming unit 200 can sequentially form light receiving images corresponding to the opening ranges and form an image of the eye E to be examined from a plurality of formed light receiving images.

The image forming unit 200 includes a processor, and realizes the function described above by performing processing according to the program(s) stored in the storage unit or the like.

In some embodiments, the light source 10 includes two or more light sources. For example, each of the two or more light sources is provided corresponding to the two or more openings formed in the iris aperture 21. In this case, the main controller 101 can change the at least one of a position of each light source or an orientation (orientation in the direction of maximum light quantity distribution) of each light source, by controlling the movement mechanisms provided for each of the two or more light sources. For example, the two or more light sources are provided corresponding to the single opening formed in the iris aperture 21. In this case, the main controller 101 can adjust the light quantity distribution of the light passing through the iris aperture 21, by independently changing the light quantity of each of the two or more light sources.

(Storage unit 102)

The storage unit 102 stores various computer programs and data. The computer programs include an arithmetic program and a control program for controlling the ophthalmic apparatus 1.

(Operation unit 110)

The operation unit 110 includes an operation device or an input device. The operation unit 110 includes buttons and switches (e.g., operation handle, operation knob, etc.) and operation devices (e.g., mouse, keyboard, etc.) provided in the ophthalmic apparatus 1. In addition, the operation unit 110 may include any operation device or any input device, such as a trackball, a control panel, a switch, a button, a dial, etc.

(Display unit 120)

The display unit 120 displays the image of the eye E to be examined generated by the image forming unit 200. The display unit 120 is configured to include a display device such as a flat panel display such as an LCD (Liquid Crystal Display). In addition, the display unit 120 may include various types of display devices such as a touch panel and the like provided in the body of the ophthalmic apparatus 1.

It should be noted that the operation unit 110 and the display unit 120 do not need to be configured to be separate devices. For example, a device like a touch panel, which has a display function integrated with an operation function, can be used. In this case, the operation unit 110 includes the touch panel and a computer program. The operation content for the operation unit 110 is fed to the controller 100 as electrical signal(s). Further, operations and inputs of information may be performed using a graphical user interface (GUI) displayed on the display unit 120 and the operation unit 110. In some embodiments, the functions of the display unit 120 and the operation unit 110 are realized a touch screen.

(Other configurations)

In some embodiments, the ophthalmic apparatus 1 further includes a fixation projection system. For example, an optical path of the fixation projection system is coupled with the optical path of the imaging optical system 40 in the configuration of the optical system shown in FIG. 1. The fixation projection system can present internal fixation targets or external fixation targets to the eye E to be examined. In case of presenting the internal fixation target to the eye E to be examined, the fixation projection system includes an LCD that displays the internal fixation target under the control from the controller 100, and projects a fixation light flux output from the LCD onto the fundus Ef of the eye E to be examined. The LCD is configured to be capable of changing the display position of the fixation target on the screen of the LCD. By changing the display position of the fixation target on the screen of the LCD, the projected position of the fixation target on the fundus of the eye E to be examined can be changed. The display position of the fixation target on the LCD can be designated using the operation unit 110 by the user.

In some embodiments, the ophthalmic apparatus 1 includes an alignment system. In some embodiments, the alignment system includes an XY alignment system and a Z alignment system. The XY alignment system is used for position matching between the optical system of the apparatus and the eye E to be examined in a direction intersecting the optical axis of the optical system of the apparatus (objective lens 46). The Z alignment system is used for position matching between the optical system of the apparatus and the eye E to be examined in a direction of the optical axis of the ophthalmic apparatus 1 (objective lens 46).

For example, the XY alignment system projects a bright spot (bright spot in the infrared region or near-infrared region) onto the eye E to be examined. The controller 100 or the image forming unit 200 acquires an anterior segment image of the eye E to be examined on which the bright spot is projected, and obtains the displacement between the bright spot image drawn on the acquired anterior segment image and an alignment reference position. The controller 100 relatively moves the optical system of the apparatus and the eye E to be examined in the direction intersecting the direction of the optical axis so as to cancel the obtained displacement, using the movement mechanism.

For example, the Z alignment system projects alignment light in infrared region or the near-infrared region from a position away from the optical axis of the optical system of the apparatus, and receives the alignment light reflected on the anterior segment of the eye E to be examined. The controller 100 or the image forming unit 200 identifies a distance to the eye E to be examined relative to the optical system of the apparatus from the light receiving position of the alignment light that changes in accordance with the distance to the eye E to be examined from the optical system of the apparatus. The controller 100 relatively moves the optical system of the apparatus and the eye E to be examined in the direction of the optical axis using the movement mechanism (not shown) so that the identified distance becomes a predetermined working distance.

In some embodiments, the function of the alignment system is realized by two or more anterior segment cameras positioned at positions away from the optical axis of the optical system of the apparatus. For example, as disclosed in Japanese Unexamined Patent Application Publication No. 2013-248376, the controller 100 or the image forming unit 200 analyzes the anterior segment images of the eye E to be examined substantially simultaneously acquired using the two or more anterior segment cameras, and identifies a three-dimensional position of the eye E to be examined using known trigonometry. The controller 100 controls the movement mechanism (not shown) to relatively move the optical system of the apparatus and the eye E to be examined three-dimensionally so that the optical axis of the optical system of the apparatus substantially coincides with an axis of the eye E to be examined and the distance of the optical system of the apparatus with respect to the eye E to be examined is a predetermined working distance.

As described above, in the ophthalmic apparatus 1, the slit 22 (opening), the photographed site (fundus Ef), and the image sensor 51 (light receiving surface) are arranged at positions substantially conjugate optically each other. The ophthalmic apparatus 1 can acquire clear images of the photographed site while suppressing the effects due to the unnecessary scattered light, by moving the light receivable range on the image sensor 51 in conjunction with the irradiated position of the illumination light.

The iris aperture 21 is an example of the "illumination diaphragm" according to the embodiments. The opening 21A formed in the iris aperture 21 is an example of the "illumination opening" according to the embodiments. The imaging optical system 40 is an example of the "light receiving optical system" according to the embodiments. The opening 45A formed in the pupil division member 45 or the photographic diaphragm included in the pupil division member 45 is an example of the "photographic diaphragm" according to the embodiments. The opening 45A formed in the pupil division member 45 is an example of the "light receiving opening" or the "photographing opening" according to the embodiments. The opening formed in the slit 22 is an example of the "slit opening" according to the embodiments.

[Operation]

Next, the operation of the ophthalmic apparatus 1 will be described.

FIG. 12 shows an example of an operation of the ophthalmic apparatus 1 according to the embodiments. FIG. 12 represents a flowchart of an example of the operation of the ophthalmic apparatus 1 according to the embodiments. The storage unit 102 stores computer program(s) for realizing the processing shown in FIG. 12. The main controller 101 operates according to the computer program, and thereby the main controller 101 performs the processing shown in FIG. 12.

Here, it is assumed that, prior to the processing shown in FIG. 12, the alignment of the optical system relative to the eye E to be examined has been completed, and the fixation target has been projected at a predetermined fixation position on the fundus Ef.

(S1: Acquire dioptric power)

First, the main controller 101 acquires the dioptric power. For example, the main controller 101 moves the focusing lens 47 to identify the in-focus state, and identifies the dioptric power from the position where the focusing lens 47, that has been set to the in-focus state, is positioned on the optical axis (or the control result for the actuator that moves the movement mechanism 47D). The main controller 101 may acquire the dioptric power of the eye E to be examined from the external ophthalmic measurement apparatus or the electronic medical record.

(S2: Change position of slit)

Next, the main controller 101 changes the position of the slit 22 on the optical axis of the illumination optical system 20 in accordance with the dioptric power of the eye E to be examined acquired in step S1.

Specifically, the main controller 101 identifies the position of the slit 22 corresponding to the dioptric power by referring to the first control information stored in the storage unit 102, and controls the movement mechanism 22D so as to dispose the slit 22 at the identified position.

(S3: Turn light source on)

Next, the main controller 101 controls the light source 10 to turn the light source 10 on, and causes the illumination optical system 20 to generate the slit-shaped illumination light. Further, the main controller 101 controls the optical scanner 30 to start the deflection control of the optical scanner 30 to start irradiating the illumination light onto a desired irradiated range on the fundus Ef. When the irradiation of the illumination light is started, the slit-shaped illumination light is sequentially irradiated within the desired irradiated range as described above.

(S4: Acquire light receiving result)

The main controller 101 acquires the light receiving result(s) of the pixels in the opening range of the image sensor 51 corresponding to the irradiated range of the illumination light on the fundus Ef performed in step S3.

(S5: Change to next position to be irradiated?)

The main controller 101 determines whether or not the irradiated position to be irradiated next with the illumination light is present. The main controller 101 can determine whether or not the next irradiated position is to be irradiated with the illumination light, by determining whether or not the irradiated range of the illumination light that is moved sequentially has covered a predetermined imaging range of the fundus Ef.

When it is determined that the irradiated position to be irradiated next with the illumination light is present (S5: Y), the operation of the ophthalmic apparatus 1 proceeds to step S6. When it is determined that the irradiated position to be irradiated next with the illumination light is not present (S5: N), the operation of the ophthalmic apparatus 1 proceeds to step S7.

(S6: Change deflection angle of illumination light)

When it is determined in step S5 that the irradiated position to be irradiated next with the illumination light is present (S5: Y), the main controller 101 controls the optical scanner 30 to change the deflection angle of the deflection surface of the optical scanner 30 by a predetermined step (angle).

Subsequently, the operation of the ophthalmic apparatus 1 proceeds to step S4. In this case, in step S4, the main controller 101 acquires the light receiving result(s) of the pixels in the opening range of the image sensor 51 corresponding to the irradiated range of the illumination light on the fundus Ef that has been moved in step S6.

(S7: Turn light source off)

When it is determined in step S5 that the irradiated position to be irradiated next with the illumination light is not present (S5: N), the main controller 101 controls the light source 10 to turn the light source 10 off. Further, the main controller 101 controls the optical scanner to stop the deflection operation of the optical scanner 30.

(S8: Form image)

Subsequently, the main controller 101 controls the image forming unit 200 to form the fundus image of the eye E to be examined from the light receiving results acquired repeatedly while changing the irradiated range of the illumination light in steps S4 to S6.

For example, the image forming unit 200 syntheses a plurality of light receiving results with different irradiated ranges (opening ranges on the light receiving surface of the image sensor 51) of the illumination light for the number of times repeating the process in steps S4 to S6, based on the order of the movement of the irradiated range. Thereby, the fundus image of the fundus Ef for one frame is formed.

In some embodiments, in step S6, the illumination light is irradiated on the irradiated range set so as to have an overlapping region with the adjacent irradiated range. Thereby, in step S8, the fundus image for one frame is formed by synthesizing the images of irradiated ranges so as to overlap the overlapping region with each other.

This terminates the operation of the ophthalmic apparatus 1 (END).

FIG. 13A and FIG. 13B show schematic explanatory diagrams in case of imaging the fundus Ef of the eye E to be examined in the ophthalmic apparatus 1 according to the embodiments. FIG. 13A schematically represents an illumination light flux and an imaging light flux in case of imaging the fundus of the eye E to be examined in the present embodiment. FIG. 13B image is an example of a fundus image of the eye E to be examined acquired in the present embodiment. In FIG. 13A, parts similar to those in FIG. 17A are denoted by the same reference symbols, and description thereof is omitted as appropriate.

As described above, the ophthalmic apparatus 1 is configured to illuminate the fundus Ef with the light, which passes through the opening 21A formed at the position eccentric to the optical axis in the iris aperture 21, and to receive the returning light, which passes through the opening 45A formed at the position eccentric to the optical axis in the pupil division member 45, from the fundus Ef. Thereby, as shown in FIG. 13A, the illumination light flux IL and the imaging light flux SL are spatially separated on the lens surface SF of the objective lens 46 and inside the lens of objective lens 46. As a result, it is possible to prevent the reflected light from the lens surface of the objective lens, which causes center ghost (objective lens surface flare), from entering an optical path of the imaging optical system 40.

Therefore, without providing a black spot plate, as shown in FIG. 13B, the acquired fundus image IMG0 does not show blocked up shadows caused by black spot shadows (that is, fundus information is not lost). Thus, the fundus image, which enables an appropriate diagnosis, of the eye to be examined can be acquired without complicating the control for the ophthalmic apparatus.

In this case, by performing pupil division so that the pupil separation amount described above becomes greater than the predetermined center ghost occurrence suppression threshold on the pupil conjugate plane, even when the eye E to be examined is a high myopia eye, the illumination light and the returning light (imaging light) can be spatially separated on the lens surface of the objective lens 46, or the like. Further, by performing pupil division so that the pupil separation amount described above becomes smaller than the predetermined small pupil photographable threshold, even when the eye E to be examined is a microcoria eye, the fundus image, which enables an appropriate diagnosis, of the eye to be examined can be acquired. Therefore, even when photographing a microcoria eye, the fundus images, which enable appropriate diagnosis for the microcoria eye, of the eye to be examined can be acquired without complicating the control for photographing the microcoria eye.

[Modification examples]

The configuration according to the embodiments is not limited to the configuration described above. The shape of each of the illumination opening and the photographing opening according to the embodiments may be any shape. Further, the number of each of the illumination opening and the photographing opening according to the embodiments may be two or more.

<First modification example>

The shape of the opening 21A formed in the iris aperture 21 according to the embodiments is not limited to the shape shown in FIG. 2. Hereinafter, the first modification example of the embodiments will be described focusing on the differences from the embodiments.

FIG. 14A shows an example of a configuration of the iris aperture 21 according to the first modification example of the embodiments. FIG. 14A schematically represents an example of the configuration of the iris aperture 21 according to the present modification example when viewed from the direction of an optical axis O. In FIG. 14A, like reference numerals designate like parts as in FIG. 2. The same description may not be repeated.

The difference between the configuration of the iris aperture 21 according to the present modification example and that of the iris aperture shown in FIG. 2 is the shape of the opening 21A. In the iris aperture 21 according to the present modification example, the opening 21A having a predetermined thickness along a circumferential direction centered on the optical axis O is formed. For example, by forming the opening 21A as shown in FIG. 14A, when the pupil center of the eye E to be examined is arranged on the optical axis O, the illumination light can enter into the eye from positions eccentric to the pupil center.

FIG. 14B shows an explanatory diagram of the pupil conjugate plane according to the first modification example of the embodiments. FIG. 14B schematically represents an image IPI1 of the illumination opening and an image SPI of the light receiving opening on the pupil conjugate plane PL. In FIG. 14B, like reference numerals designate like parts as in FIG. 4. The same description may not be repeated.

By performing pupil division with the pupil division member 45, the image IPI1 of the illumination opening, that is an image of the opening formed in the iris aperture 21 according to the present modification example, and the image SPI of the light receiving opening, that is an image of the opening formed in the photographic diaphragm (pupil division member 45) are spatially divided on the pupil conjugate plane PL. In this case, the image IPI1 of the illumination opening and the image SPI of the light receiving opening are divided by a pupil separation amount GP. Here, the pupil separation amount GP corresponds to the shortest distance in the arrangement direction (separation direction) between the image IPI1 of the illumination opening and the image SPI of the light receiving opening.

The pupil separation amount GP on the pupil conjugate plane according to the present modification example equals the pupil separation amount GP shown in FIG. 4. Also in the present modification example, by increasing the pupil separation amount GP, the overlap of the light flux cross-sections between the illumination light and the returning light on the lens surface of the objective lens 46 or inside the objective lens 46 can be reliably avoided. As a result, the occurrence of center ghost can be suppressed without requiring a black spot plate. Therefore, also in the present modification example, it is desirable that the opening 21A (or one or more openings) is formed in the iris aperture 21 and the opening 45A (or one or more openings) is formed in the pupil division member 45 so that the pupil separation amount GP becomes greater than the predetermined center ghost occurrence suppression threshold.

On the other hand, when the pupil separation amount GP is increased, the size of the pupil diameter that can be imaged becomes larger, which makes it impossible to photograph a microcoria eyes. Therefore, also in the present modification example, it is desirable that the opening 21A (or one or more openings) is formed in the iris aperture 21 and the opening 45A (or one or more openings) is formed in the pupil division member 45 so that the pupil separation amount GP becomes smaller than the predetermined small pupil photographable threshold.

Additionally, the opening 21A formed in the iris aperture 21 may have a circular segment shape. Here, the circular segment is a region bounded by an inferior arc of a circle or ellipse and a chord of this inferior arc. A direction of the chord of the circular segment shape is approximately parallel to a direction corresponding to the slit direction of the opening(s) formed in the slit 22.

<Second modification example>

The number of the openings formed in the iris aperture 21 according to the embodiments is not limited to one. Hereinafter, the second modification example of the embodiments will be described focusing on the differences from the embodiments.

FIG. 15 shows an example of a configuration of the iris aperture 21 according to the second modification example of the embodiments. FIG. 15 schematically represents an example of the configuration of the iris aperture 21 according to the present modification example when viewed from the direction of the optical axis O. In FIG. 15, the same parts as in FIG. 2 are shown by the same symbols and their descriptions will be omitted in an appropriate manner.

The difference between the configuration of the iris aperture 21 according to the present modification example and that of the iris aperture shown in FIG. 2 is the number of the openings. In the iris aperture 21 according to the present modification example, the openings 21A and 21B are formed at position(s) eccentric to the optical axis O. For example, by forming the openings 21A and 21B as shown in FIG. 15, when the pupil center of the eye E to be examined is arranged on the optical axis O, the illumination light can enter into the eye from positions eccentric to the pupil center.

The pupil separation amount GP on the pupil conjugate plane according to the present modification example equals the pupil separation amount GP shown in FIG. 4. Also in the present modification example, by increasing the pupil separation amount GP, the overlap of the light flux cross-sections between the illumination light and the returning light on the lens surface of the objective lens 46 or inside the objective lens 46 can be reliably avoided. As a result, the occurrence of center ghost can be suppressed without requiring a black spot plate. Therefore, also in the present modification example, it is desirable that the openings 21A and 21B are formed in the iris aperture 21 and the opening 45A (or one or more openings) is formed in the pupil division member 45 so that the pupil separation amount GP becomes greater than the predetermined center ghost occurrence suppression threshold.

On the other hand, when the pupil separation amount GP is increased, the size of the pupil diameter that can be imaged becomes larger, which makes it impossible to photograph a microcoria eyes. Therefore, also in the present modification example, it is desirable that the openings 21A and 21B are formed in the iris aperture 21 and the opening 45A (or one or more openings) is formed in the pupil division member 45 so that the pupil separation amount GP becomes smaller than the predetermined small pupil photographable threshold.

<Third modification example>

The number of the openings formed in the pupil division member 45 according to the embodiments is not limited to one. Hereinafter, the third modification example of the embodiments will be described focusing on the differences from the embodiments.

FIG. 16 shows an example of a configuration of the pupil division member 45 according to the third modification example of the embodiments. FIG. 16 schematically represents the pupil division member 45 according to the present modification example, by corresponding a front view when viewing from the direction of the optical axis O with a side view when viewing form a direction perpendicular to the optical axis O. In FIG. 16, the same parts as in FIG. 3 are shown by the same symbols and their descriptions will be omitted in an appropriate manner.

The difference between the configuration of the pupil division member 45 according to the present modification example and that of the pupil division member 45 shown in FIG. 3 is the number of the openings. In the pupil division member 45 according to the present modification example, the openings 45A and 45B are formed at positions eccentric to the optical axis O. For example, by forming the openings 45A and 45B as shown in FIG. 16, when the pupil center of the eye E to be examined is arranged on the optical axis O, the returning light of the illumination light that enters into the eye from positions eccentric to the pupil center can be guided to the image sensor 51.

The pupil separation amount GP on the pupil conjugate plane according to the present modification example equals the pupil separation amount GP shown in FIG. 4. Also in the present modification example, by increasing the pupil separation amount GP, the overlap of the light flux cross-sections between the illumination light and the returning light on the lens surface of the objective lens 46 or inside the objective lens 46 can be reliably avoided. As a result, the occurrence of center ghost can be suppressed without requiring a black spot plate. Therefore, also in the present modification example, it is desirable that the opening 21A (or one or more openings) is formed in the iris aperture 21 and the openings 45A and 45B are formed in the pupil division member 45 so that the pupil separation amount GP becomes greater than the predetermined center ghost occurrence suppression threshold.

On the other hand, when the pupil separation amount GP is increased, the size of the pupil diameter that can be imaged becomes larger, which makes it impossible to photograph a microcoria eyes. Therefore, also in the present modification example, it is desirable that the opening 21A (or one or more openings) is formed in the iris aperture 21 and the openings 45A and 45B are formed in the pupil division member 45 so that the pupil separation amount GP becomes smaller than the predetermined small pupil photographable threshold.

<Other modification examples>

The illumination optical system 20 may include a projector with a light source, and the projector may emit the slit-shaped illumination light. In this case, the projector is provided instead of the light source 10 and the slit 22 in FIG. 1. Examples of the projector include an LCD (Liquid Crystal Display) type projector using a transmissive LCD panel, an LCOS (Liquid Crystal On Silicon) type projector using a reflective LCD panel, a DLP (Digital Light Processing) (registered trademark) type projector using a DMD (Digital Mirror Device). For example, the illumination optical system 20 may be configured to irradiate the slit-shaped illumination emitted by the projector onto the iris aperture 21.

[Actions]

The ophthalmic apparatus according to the embodiments will be described.

The first aspect of the embodiments is an ophthalmic apparatus (1). The ophthalmic apparatus includes an objective lens (46), an illumination optical system (20), an optical scanner (30), a light receiving optical system (imaging optical system 40), and a pupil division member (45). The illumination optical system includes an illumination diaphragm (iris aperture 21) with one or more openings (21A, 21B) formed at positions eccentric to an optical axis (O). The illumination diaphragm is arranged at a position substantially conjugate optically to a pupil (Eu) of an eye (E) to be examined. The illumination optical system is configured to irradiate slit-shaped illumination light. The optical scanner is arranged at a position substantially conjugate optically to the pupil, and is configured to deflect the illumination light to guide the deflected illumination light to the eye to be examined via the objective lens. The light receiving optical system is configured to guide returning light of the illumination light from the eye to be examined to an image sensor (51). The returning light passes through the objective lens. The image sensor is arranged at a position substantially conjugate optically to a photographed site of the eye to be examined. The pupil division member includes a photographic diaphragm with one or more openings (45A, 45B) formed at positions eccentric to an optical axis. The photographic diaphragm is arranged at a position substantially conjugate optically to the pupil. The pupil division member is configured to spatially separate an optical path of the illumination optical system and an optical path of the light receiving optical system.

According to such an aspect, the illumination light and the returning light can be spatially separated on the lens surface of the objective lens and inside the lens of the objective lens. As a result, it is possible to prevent the reflected light from the lens surface of the objective lens, which causes center ghost (objective lens surface flare), from entering an optical path of the light receiving optical system. Therefore, it becomes no longer necessary to provide a black spot plate, which allows to acquire images of the eye to be examined without blocked up shadows caused to the black spot shadow (without loss of intraocular information such as fundus information). Further, even when photographing a microcoria eye (eye with small pupil), intraocular images, that enable appropriate diagnosis for the microcoria eye, of the eye to be examined can be acquired without complicating the control for photographing the microcoria eye.

In the second aspect of the embodiments, in the first aspect, the illumination optical system includes a slit (22) with a slit-shaped opening, the slit being arranged at a position substantially conjugate optically to the photographed site, and is configured to irradiate light from a light source onto the slit.

According to such an aspect, the slit-shaped illumination can be irradiated with a simple configuration. Therefore, the image of the eye to be examined without blocked up shadows caused to the black spot shadow (without loss of intraocular information such as fundus information) can be acquired without providing a black spot plate, with a simple configuration.

In the third aspect of the embodiments, in the first aspect or the second aspect, the pupil division member further includes a reflection member (45b) configured to deflect the illumination light deflected by the optical scanner toward the eye to be examined.

According to such an aspect, the configuration of the pupil division member can be further simplified.

In the fourth aspect of the embodiments, in the first aspect or the second aspect, the pupil division member is a hole mirror with a reflection member provided around an opening formed in the photographic diaphragm.

According to such an aspect, the function of the pupil division member can be realized using the hole mirror having a simple configuration.

In the fifth aspect of the embodiments, in any one of the first aspect to the fourth aspect, the image sensor is configured to capture a light receiving result in a virtual opening region on a receiving surface using a rolling shutter method, the virtual opening region corresponding to an irradiated region of the illumination light on the photographed site, the irradiated region being moved in a predetermined scan direction by the optical scanner.

According to such an aspect, the effect of unnecessary scattered light can be eliminated reliably, and the image quality of the image of the eye to be examined can be further improved.

In the sixth aspect of the embodiments, in any one of the first aspect to the fifth aspect, the image sensor is a CMOS image sensor.

According to such an aspect, it is possible to prevent the reflected light from the lens surface of the objective lens, which causes center ghost (objective lens surface flare), from entering an optical path of the light receiving optical system, with a simple configuration and low cost.

The seventh aspect of the embodiments, in any one of the first aspect to the sixth aspect, further includes an image forming unit (200) configured to form an image of the eye to be examined based on a light receiving result on a light receiving surface of the image sensor.

According to such an aspect, the image of the eye to be examined without blocked up shadows caused to the black spot shadow can be acquired without providing a black spot plate. Further, even when photographing a microcoria eye (eye with small pupil), intraocular images, that enable appropriate diagnosis for the microcoria eye, of the eye to be examined can be acquired without complicating the control for photographing the microcoria eye.

In the eighth aspect of the embodiments, in any one of the first aspect to the seventh aspect, the photographed site is a fundus (Ef).

According to such an aspect, fundus image of the eye to be examined without blocked up shadows caused to the black spot shadow can be acquired without providing a black spot plate. Further, even when photographing a microcoria eye (eye with small pupil), fundus images, that enable appropriate diagnosis for the microcoria eye, of the eye to be examined can be acquired without complicating the control for photographing the microcoria eye.

In the ninth aspect of the embodiments, in any one of the first aspect to the eighth aspect, a single opening (21A) is formed in the illumination diaphragm, and a single opening (45A) is formed in the photographic diaphragm.

According to such an aspect, it is possible to prevent the reflected light from the lens surface of the objective lens, which causes center ghost, from entering an optical path of the light receiving optical system, with a simple configuration, using the single illumination opening and the single photographing opening that are formed at positions eccentric to the optical axis.

In the tenth aspect of the embodiments, in any one of the first aspect to the eighth aspect, the one or more openings are formed in the illumination diaphragm and the one or more openings are formed in the photographic diaphragm so that a pupil separation amount (GP) becomes greater than a predetermined center ghost occurrence suppression threshold, the pupil separation amount being a shortest distance in an arrangement direction of images of the one or more openings in the illumination diaphragm and images of the one or more opening formed in the photographic diaphragm on a pupil conjugate plane (PL) at a position substantially conjugate optically to the pupil.

According to such an aspect, the occurrence of center ghost can be reliably suppressed without providing a black spot plate.

In the eleventh aspect of the embodiments, in the tenth aspect, the one or more openings are formed in the illumination diaphragm and the one or more openings are formed in the photographic diaphragm so that the pupil separation amount is approximately constant in a direction perpendicular to the arrangement direction. Here, the arrangement direction is a direction in which the images of the one or more openings formed in the illumination diaphragm and the images of the one or more opening formed in the photographic diaphragm are arranged on the pupil conjugate plane.

According to such an aspect, the pupil separation amount can be maximized while ensuring light quantity by increasing the size of the opening.

In the twelfth aspect of the embodiments, in the tenth aspect, the predetermined center ghost occurrence suppression threshold is determined in accordance with a working distance (WD) of the ophthalmic apparatus, a dioptric power (D) of an eye to be examined that can be imaged, a viewing angle ($\alpha$) of the illumination light with respect to the photographed site, and a viewing angle ($\gamma$) of the returning light with respect to the fundus (Ef).

According to such an aspect, the occurrence of center ghost can be reliably suppressed, by the working distance of the ophthalmic apparatus, the dioptric power of the eye to be examined that can be imaged, the viewing angle of the illumination light with respect to the photographed site, and the viewing angle of the returning light with respect to the fundus.

In the thirteenth aspect of the embodiments, in the twelfth aspect, the dioptric power is a dioptric power of a high myopia eye.

According to such an aspect, even if the eye to be examined is a high myopia aye, the occurrence of the center ghost can be reliably suppressed without providing a black spot plate.

In the fourteenth aspect of the embodiments, in the twelfth aspect, the one or more openings are formed in the illumination diaphragm and the one or more openings are formed in the photographic diaphragm so that the pupil separation amount becomes smaller than a predetermined small pupil photographable threshold.

According to such an aspect, even if the eye to be examined is a microcoria eye, the occurrence of the center ghost can be is reliably suppressed without providing a black spot plate.

In the fifteenth aspect of the embodiments, in the fourteenth aspect, the predetermined small pupil photographable threshold is determined in accordance with a predetermined pupil diameter for small pupil photography, a width in the arrangement direction of the images of the one or more openings formed in the illumination diaphragm, and a width in the arrangement direction of the images of the one or more openings formed in the photographic diaphragm. Here, the arrangement direction is a direction in which the images of the one or more openings formed in the illumination diaphragm and the images of the one or more opening formed in the photographic diaphragm are arranged on the pupil conjugate plane.

According to such an aspect, the microcoria eye can be reliably imaged, by determining the predetermined pupil diameter for small pupil photography, the width in the arrangement direction of the images of the one or more openings formed in the illumination diaphragm, and the width in the arrangement direction of the images of the one or more openings formed in the photographic diaphragm.

The embodiments or the modification examples thereof described above are merely examples for carrying out the present invention. Those who intend to implement the present invention can apply any modification, omission, addition, or the like within the scope of the gist of the present invention.

In the above embodiments, the ophthalmic apparatus may have arbitrary functions adaptable in the field of ophthalmology. Examples of such functions include an axial length measurement function, a tonometry function, an optical coherence tomography (OCT) function, an ultrasonic inspection, and the like. It should be noted that the axial length measurement function is realized by the OCT, etc. Further, the axial length measurement function may be used to measure the axial length of the eye to be examined by projecting light onto the eye to be examined and detecting the returning light from the fundus while adjusting the position of the optical system in the Z direction (front-back direction) relative to the eye to be examined. The intraocular pressure measurement function is realized by the tonometer, etc. The OCT function is realized by the OCT apparatus, etc. The ultrasonic inspection function is realized by the ultrasonic diagnosis apparatus, etc. Further, the present invention can also be applied to an apparatus (multifunctional apparatus) having two or more of such functions.

EXPLANATION OF SYMBOLS

| | |
|---|---|
| 1 | Ophthalmic apparatus |
| 10 | Light source |
| 20 | Illumination optical system |
| 21 | Iris aperture |
| 22 | Slit |
| 30 | Optical scanner |
| 35 | Projection optical system |
| 40 | Imaging optical system |
| 41, 43, 48 | Relay lens |
| 42 | Reflective mirror |
| 45 | Pupil division member |
| 46 | Objective lens |
| 47 | Focusing lens |
| 49 | Imaging lens |
| 50 | Imaging device |
| 51 | Image sensor |
| 100 | Controller |
| 101 | Main controller |
| 102 | Storage unit |
| 200 | Image forming unit |
| E | Eye to be examined |
| Ef | Fundus |
| Eu | Pupil |

## Claims

**1.** An ophthalmic apparatus, comprising:

an objective lens;
an illumination optical system including an illumination diaphragm with one or more openings formed at positions eccentric to an optical axis, the illumination diaphragm being arranged at a position substantially conjugate optically to a pupil of an eye to be examined, and configured to irradiate slit-shaped illumination light;
an optical scanner arranged at a position substantially conjugate optically to the pupil, and configured to deflect the illumination light to guide the deflected illumination light to the eye to be examined via the objective lens;
a light receiving optical system configured to guide returning light of the illumination light from the eye to be examined to an image sensor, the returning light passing through the objective lens, the image sensor being arranged at a position substantially conjugate optically to a photographed site of the eye to be examined; and
a pupil division member including a photographic diaphragm with one or more openings formed at positions eccentric to an optical axis, the photographic diaphragm being arranged at a position substantially conjugate optically to the pupil, and configured to spatially separate an optical path of the illumination optical system and an optical path of the light receiving optical system.

**2.** The ophthalmic apparatus of claim 1, wherein
the illumination optical system includes a slit with a slit-shaped opening, the slit being arranged at a position substantially conjugate optically to the photographed site, and is configured to irradiate light from a light source onto the slit.

**3.** The ophthalmic apparatus of claim 1, wherein
the pupil division member further includes a reflection member configured to deflect the illumination light deflected by the optical scanner toward the eye to be examined.

**4.** The ophthalmic apparatus of claim 1, wherein
the pupil division member is a hole mirror with a reflection member provided around an opening formed in the photographic diaphragm.

**5.** The ophthalmic apparatus of claim 1, wherein
the image sensor is configured to capture a light receiving result in a virtual opening region on a light receiving surface using a rolling shutter method, the virtual opening region corresponding to an irradiated region of the illumination light on the photographed site, the irradiated region being moved in a predetermined scan direction by the optical scanner.

**6.** The ophthalmic apparatus of claim 5, wherein
the image sensor is a complementary metal-oxide-semiconductor image sensor.

**7.** The ophthalmic apparatus of claim 1, further comprising
an image forming unit configured to form an image of the eye to be examined based on a light receiving result on a light receiving surface of the image sensor.

**8.** The ophthalmic apparatus of claim 1, wherein
the photographed site is a fundus.

**9.** The ophthalmic apparatus of any one of claims 1 to 8, wherein

a single opening is formed in the illumination diaphragm, and
a single opening is formed in the photographic diaphragm.

**10.** The ophthalmic apparatus of any one of claims 1 to 8, wherein
the one or more openings are formed in the illumination diaphragm and the one or more openings are formed in the photographic diaphragm so that a pupil separation amount becomes greater than a predetermined center ghost occurrence suppression threshold, the pupil separation amount being a shortest distance in an arrangement direction of images of the one or more openings in the illumination diaphragm and images of the one or more opening formed in the photographic diaphragm on a pupil conjugate plane at a position substantially conjugate optically to the pupil.

**11.** The ophthalmic apparatus of claim 10, wherein
the one or more openings are formed in the illumination diaphragm and the one or more openings are formed in the photographic diaphragm so that the pupil separation amount is approximately constant in a direction perpendicular to the arrangement direction.

**12.** The ophthalmic apparatus of claim 10, wherein
the predetermined center ghost occurrence suppression threshold is determined in accordance with a working distance of the ophthalmic apparatus, a dioptric power of an eye to be examined that can be imaged, a viewing angle of the illumination light with respect to the photographed site, and a viewing angle of the returning light with respect to the fundus.

**13.** The ophthalmic apparatus of claim 12, wherein
the dioptric power is a dioptric power of a high myopia eye.

**14.** The ophthalmic apparatus of claim 12, wherein
the one or more openings are formed in the illumination diaphragm and the one or more openings are formed in the photographic diaphragm so that the pupil separation amount becomes smaller than a predetermined small pupil photographable threshold.

**15.** The ophthalmic apparatus of claim 14, wherein
the predetermined small pupil photographable threshold is determined in accordance with a predetermined pupil diameter for small pupil photography, a width in the arrangement direction of the images of the one or more openings formed in the illumination diaphragm, and a width in the arrangement direction of the images of the one or more openings formed in the photographic diaphragm.

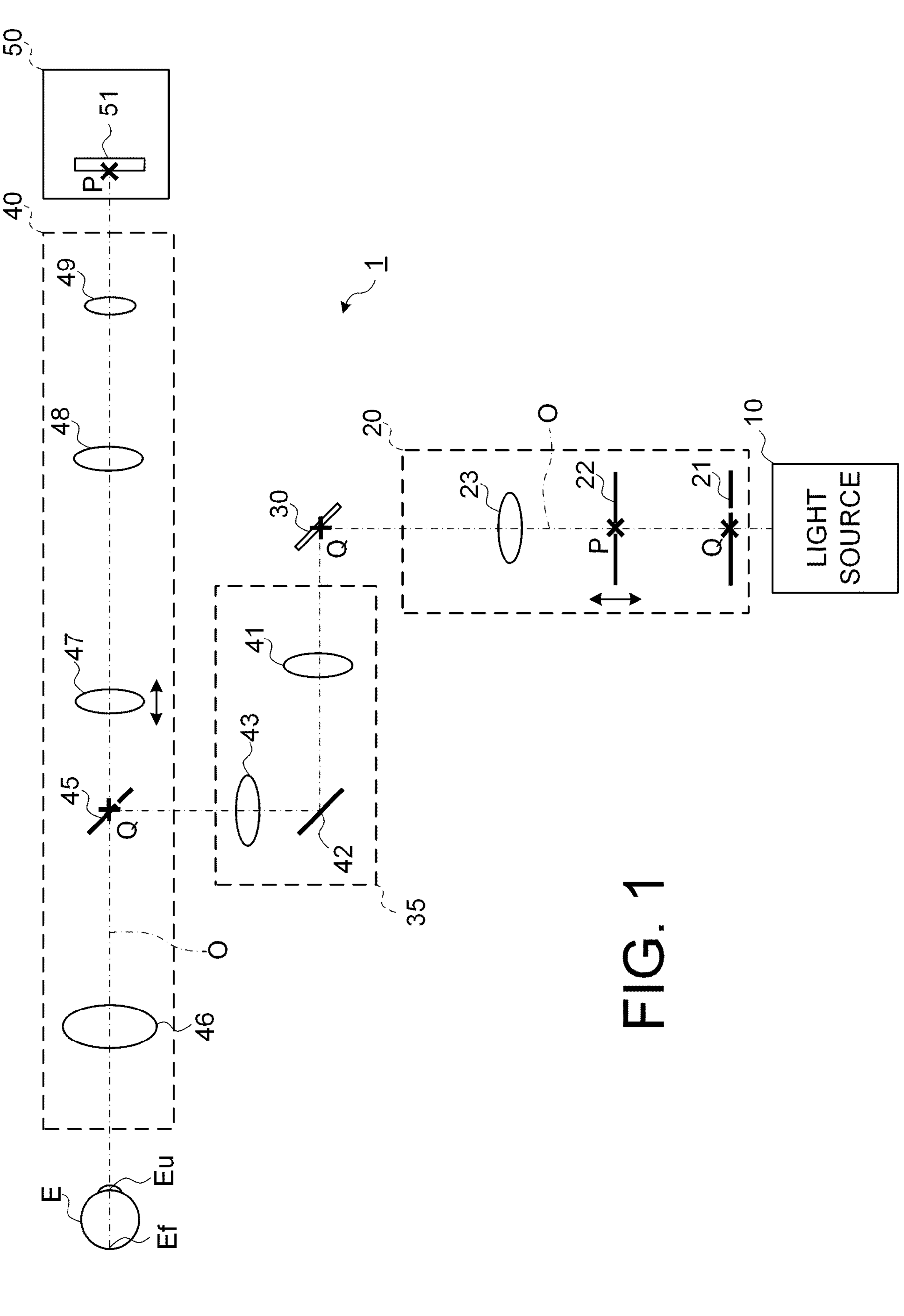
50
51
P
40
49
1
48
20
O
10
23
22
21
30
Q
P
Q
LIGHT SOURCE
47
41
43
45
Q
42
35
O
46
E
Eu
Ef

FIG. 1

FIG. 2

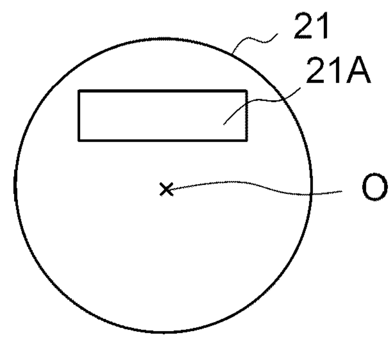


FIG. 3

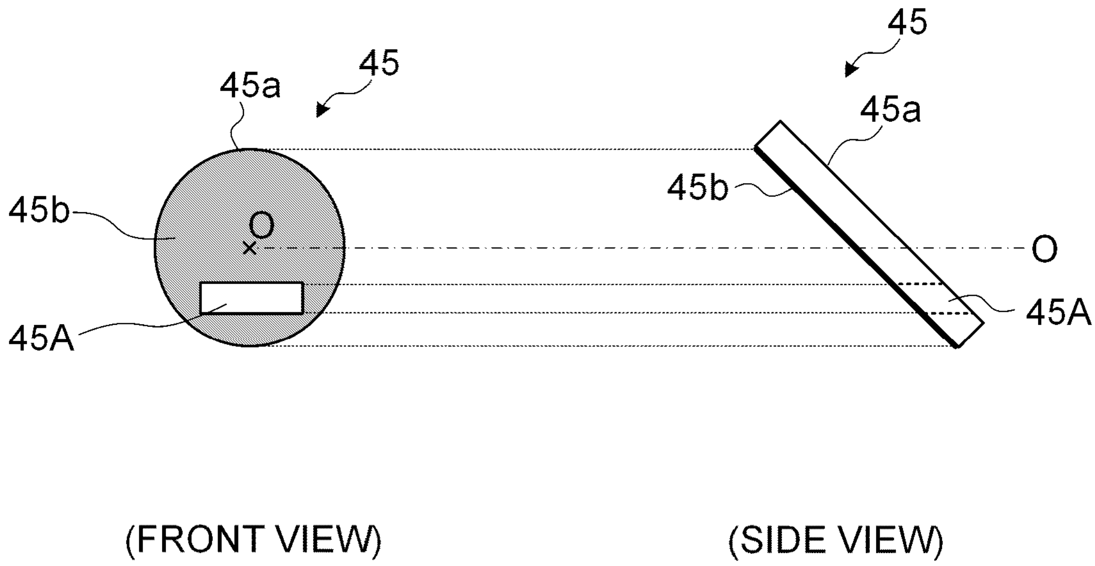

FIG. 4

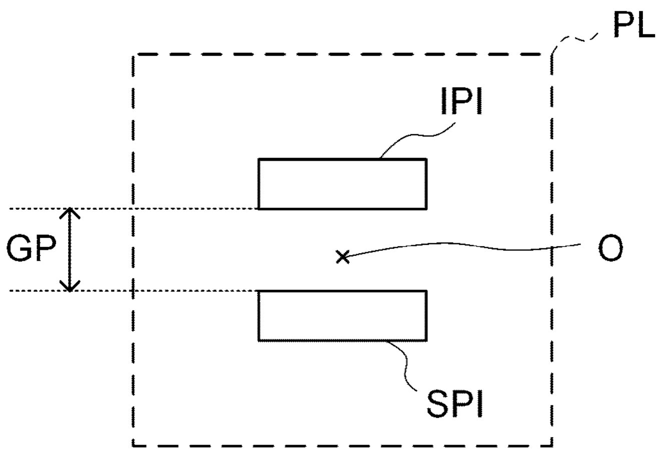


FIG. 5

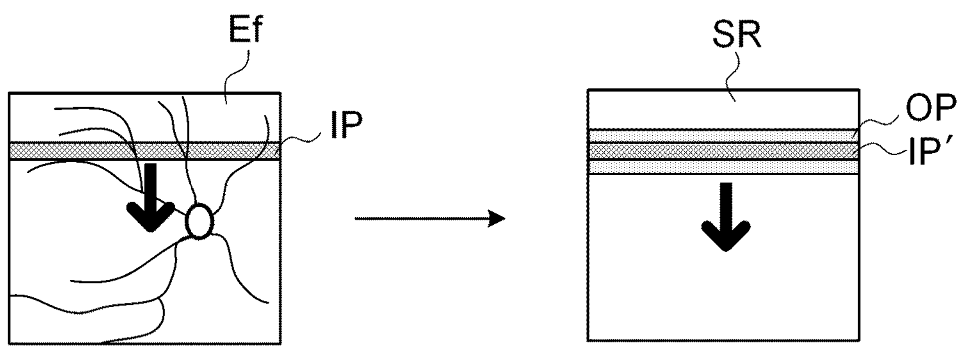

FIG. 6

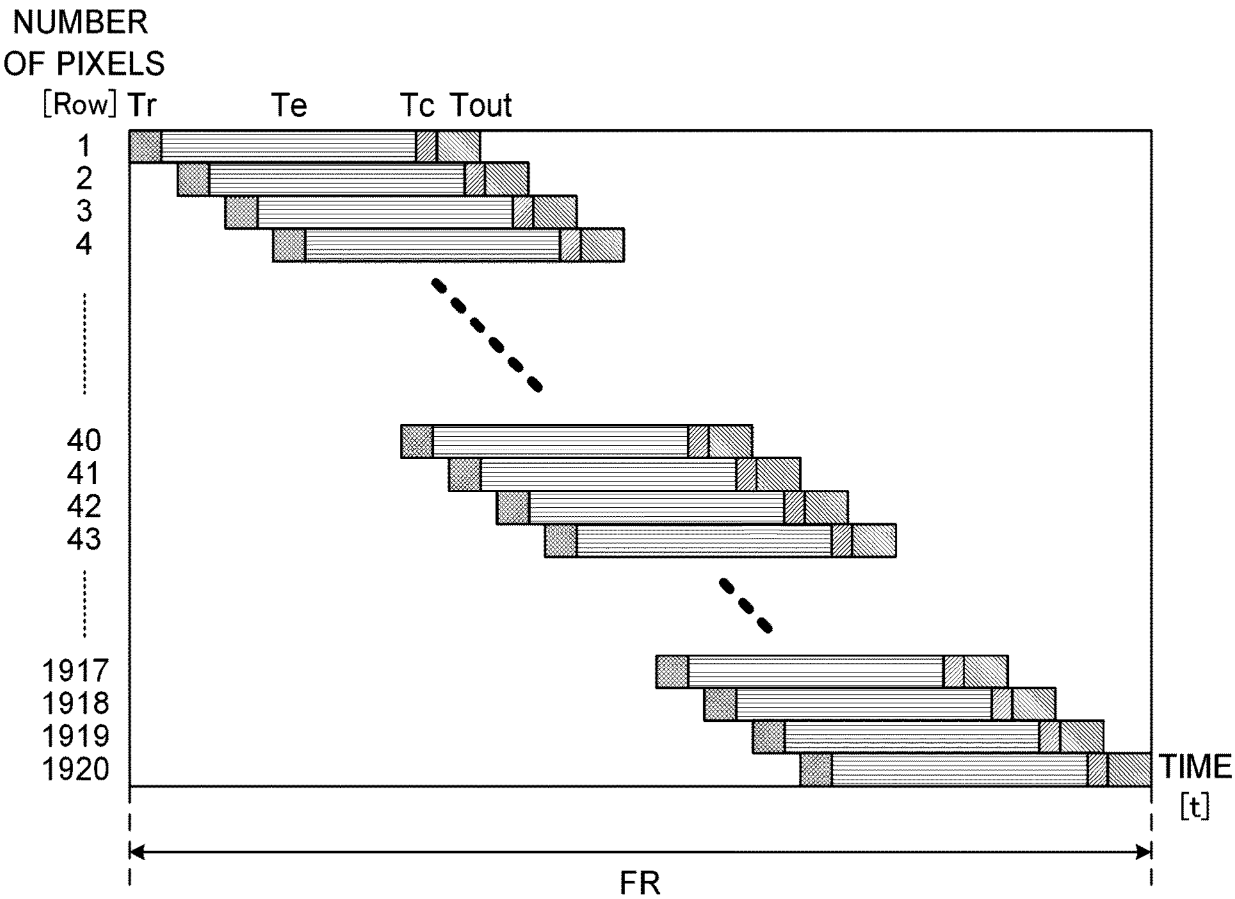
NUMBER
OF PIXELS
[Row]
Tr
Te
Tc
Tout
1
2
3
4
40
41
42
43
1917
1918
1919
1920
TIME
[t]
FR

FIG. 7

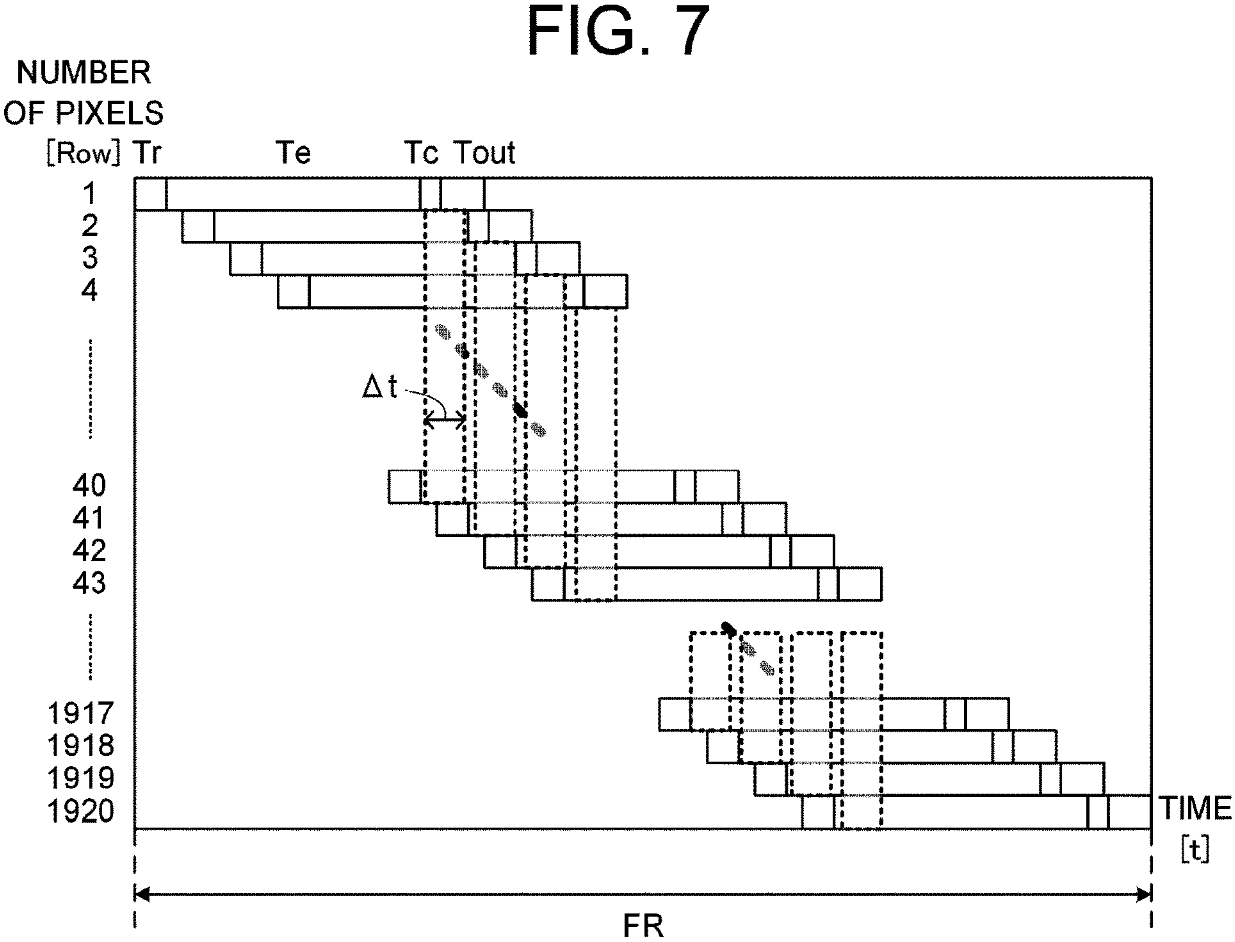
NUMBER
OF PIXELS
[Row]
Tr
Te
Tc
Tout
1
2
3
4
Δt
40
41
42
43
1917
1918
1919
1920
TIME
[t]
FR

FIG. 8

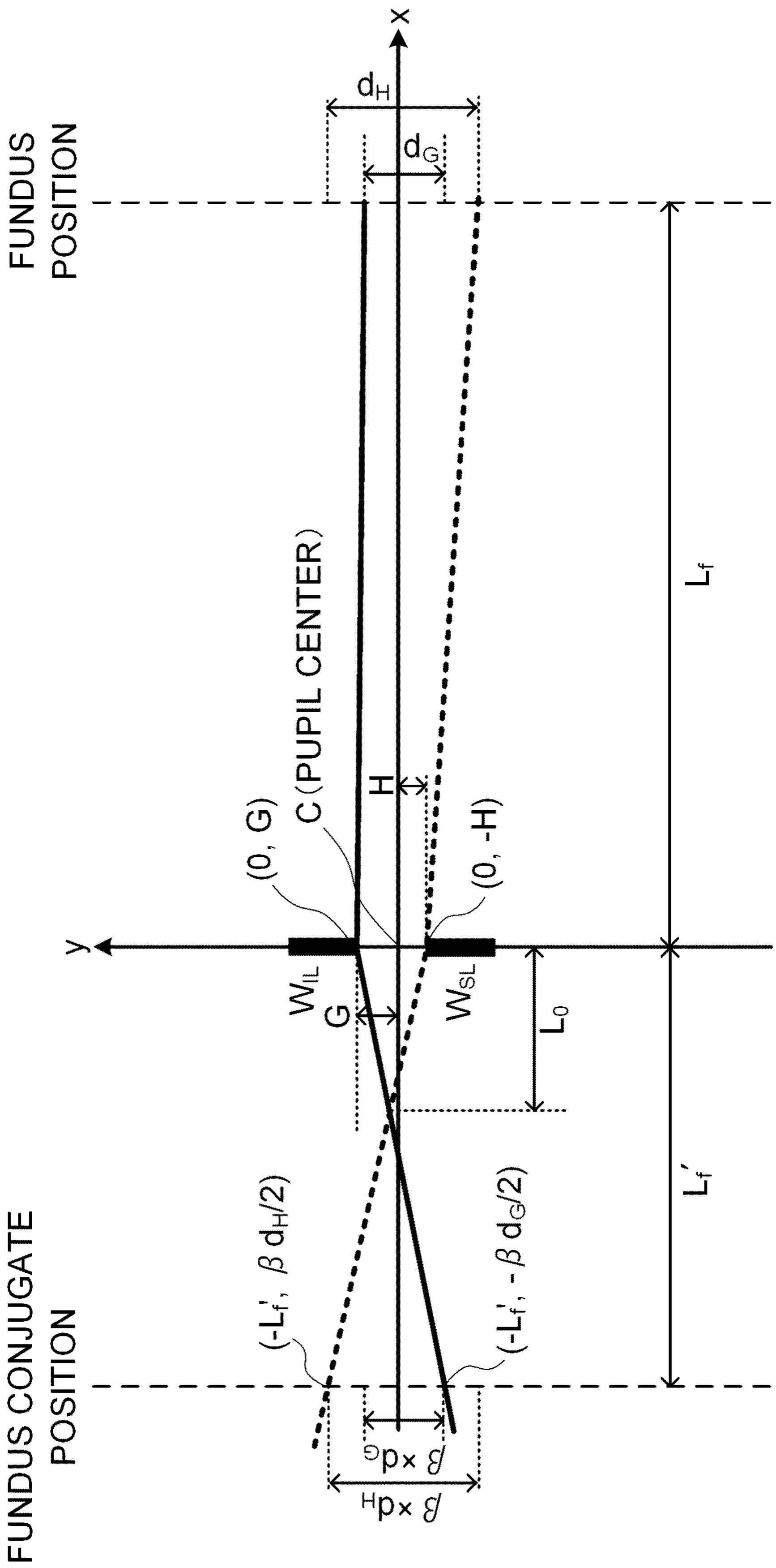
FUNDUS POSITION
FUNDUS CONJUGATE POSITION
x
y
$d_H$
$d_G$
C（PUPIL CENTER）
(0, G)
(0, -H)
H
G
$W_{IL}$
$W_{SL}$
$L_0$
$L_f$
$L_f'$
$(-L_f', \beta d_H/2)$
$(-L_f', -\beta d_G/2)$
$\beta \times d_G$
$\beta \times d_H$

FIG. 9

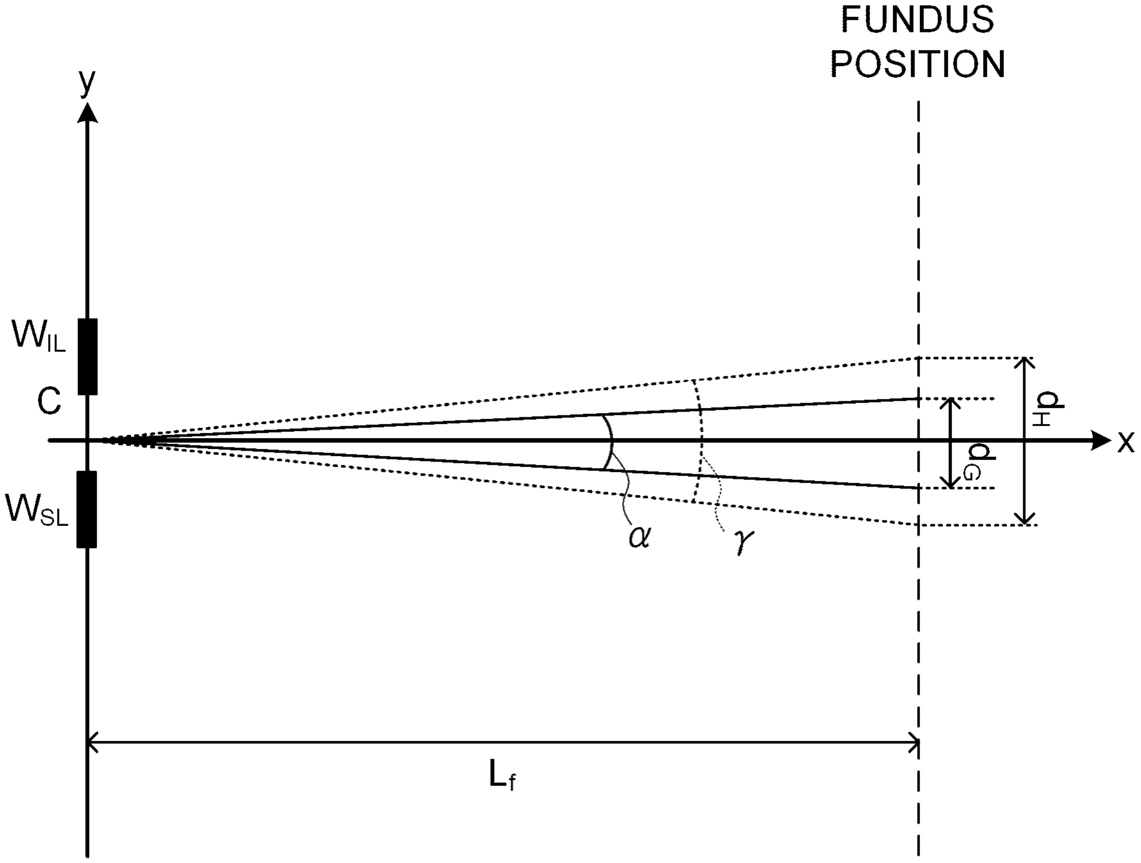


FIG. 10

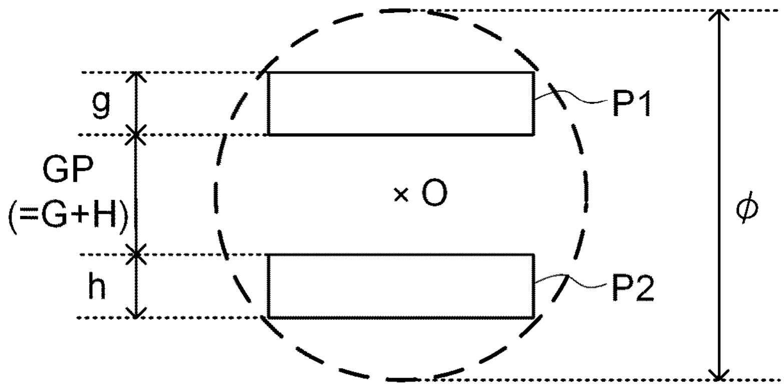

FIG. 11

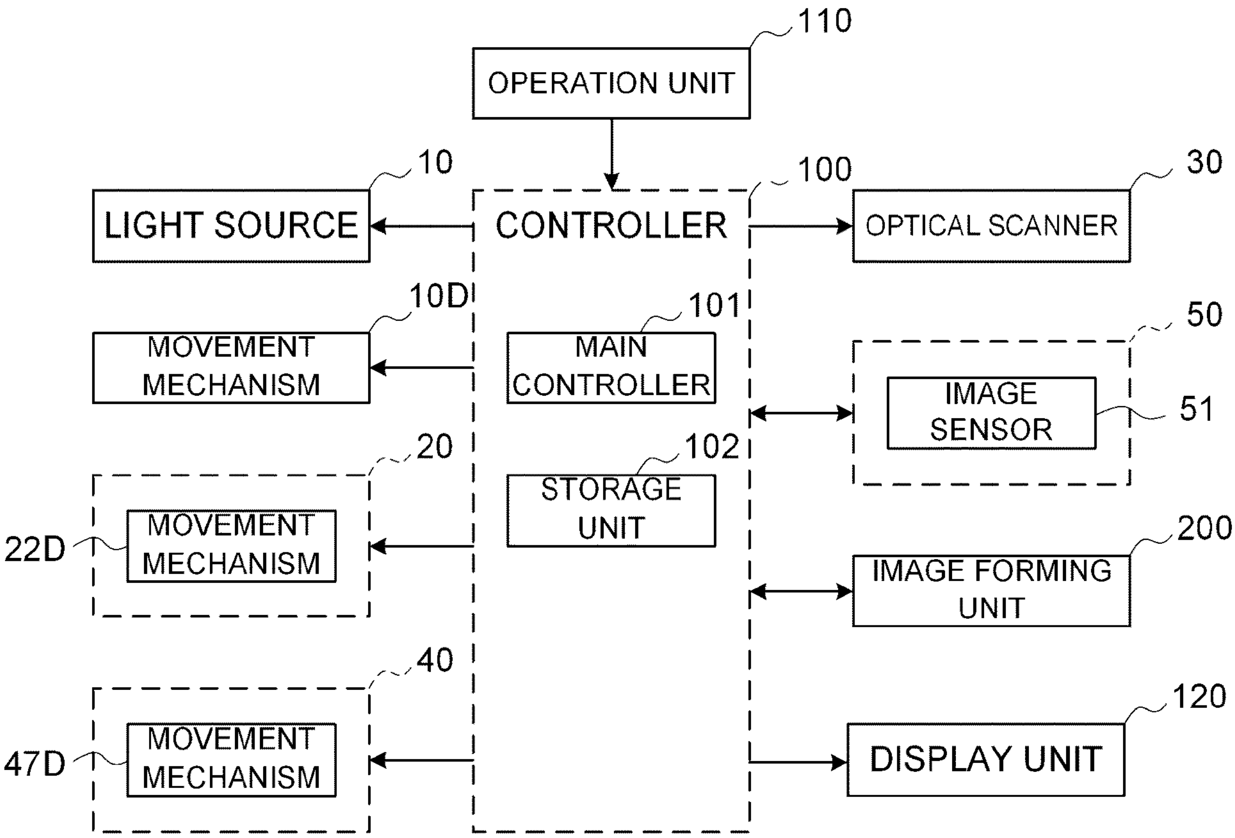
110
OPERATION UNIT
10
LIGHT SOURCE
100
CONTROLLER
30
OPTICAL SCANNER
10D
MOVEMENT MECHANISM
101
MAIN CONTROLLER
50
IMAGE SENSOR
51
20
22D
MOVEMENT MECHANISM
102
STORAGE UNIT
200
IMAGE FORMING UNIT
40
47D
MOVEMENT MECHANISM
120
DISPLAY UNIT

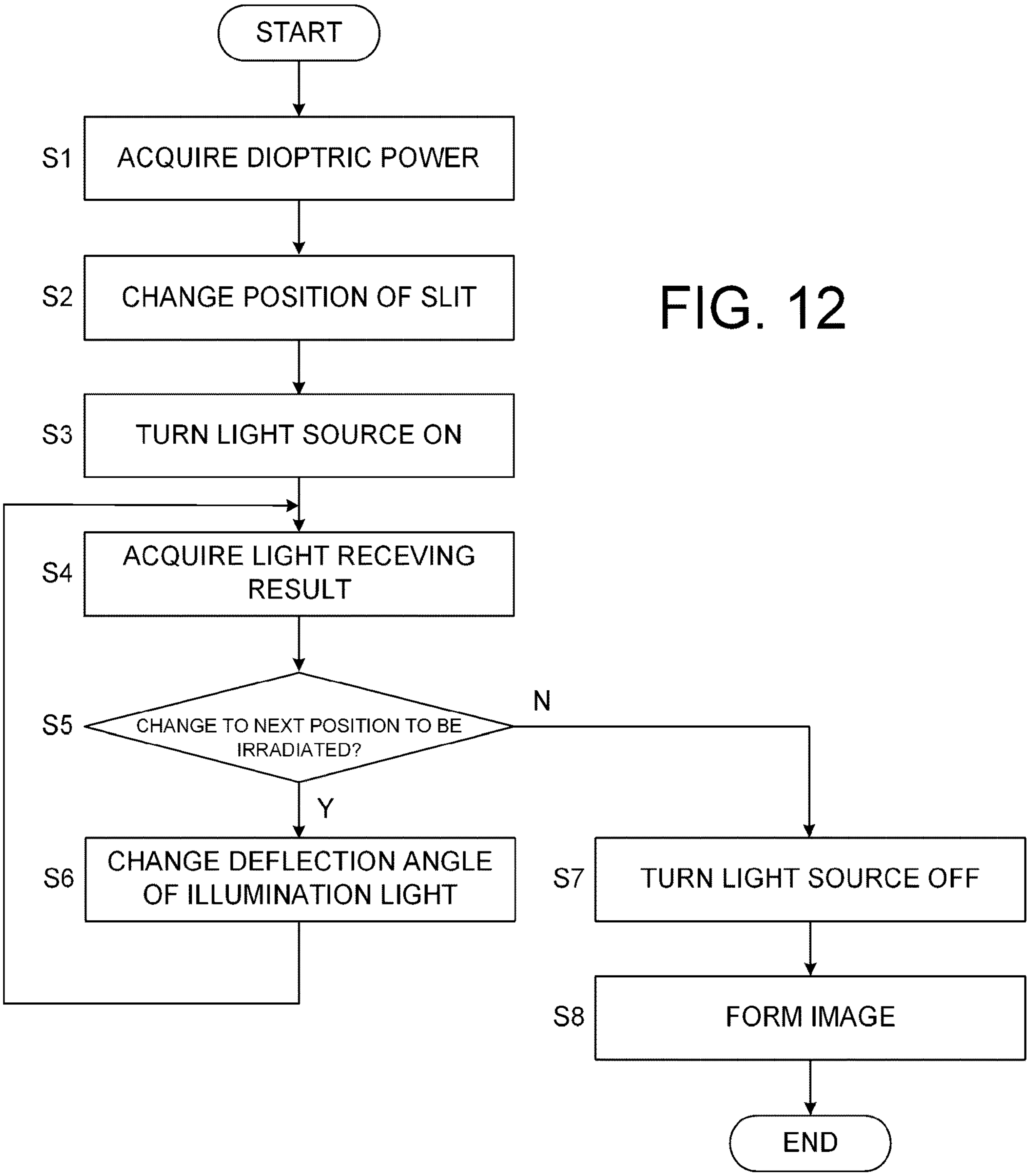
START
S1
ACQUIRE DIOPTRIC POWER
S2
CHANGE POSITION OF SLIT
S3
TURN LIGHT SOURCE ON
S4
ACQUIRE LIGHT RECEVING RESULT
S5
CHANGE TO NEXT POSITION TO BE IRRADIATED?
N
Y
S6
CHANGE DEFLECTION ANGLE OF ILLUMINATION LIGHT
S7
TURN LIGHT SOURCE OFF
S8
FORM IMAGE
END

FIG. 12

FIG. 13A

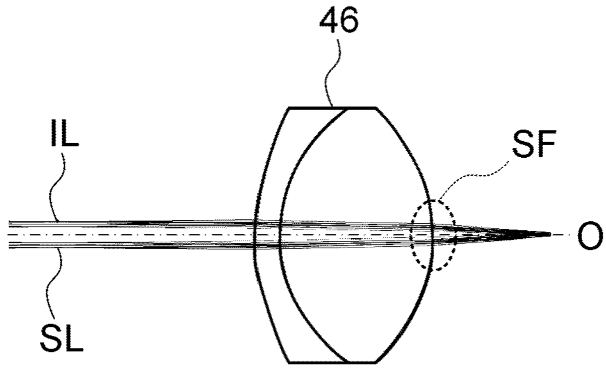


FIG. 13B

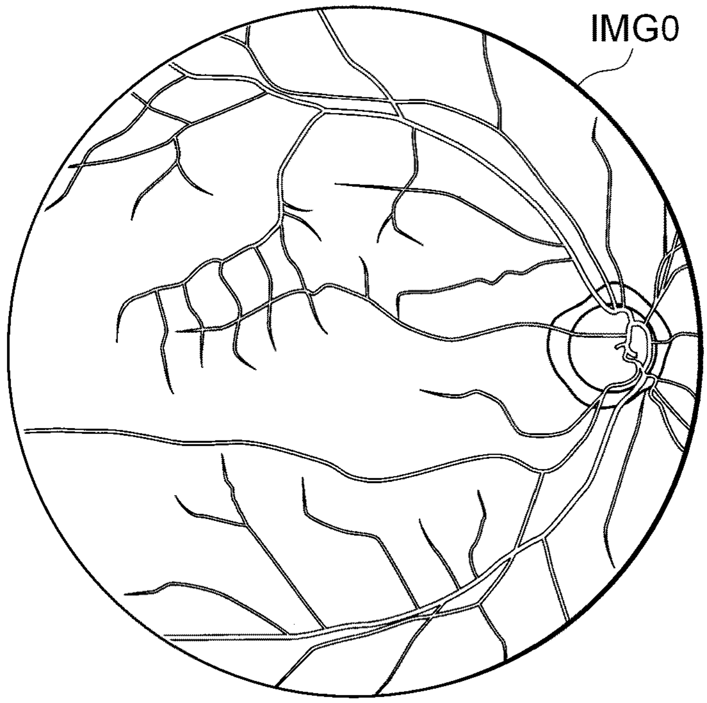

FIG. 14A

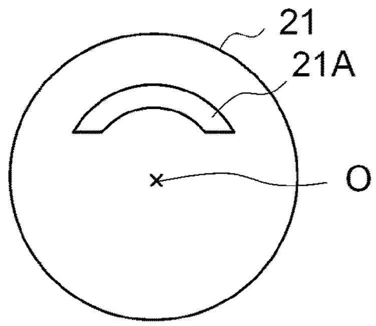


FIG. 14B

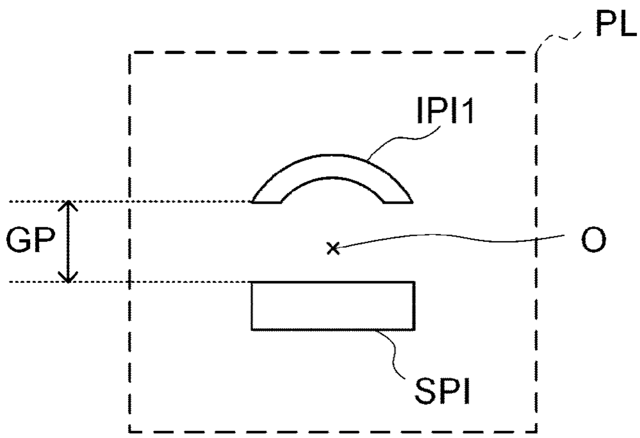


FIG. 15

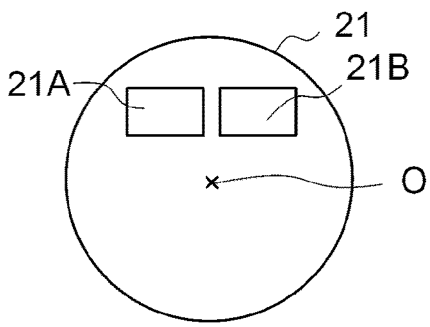

FIG. 16

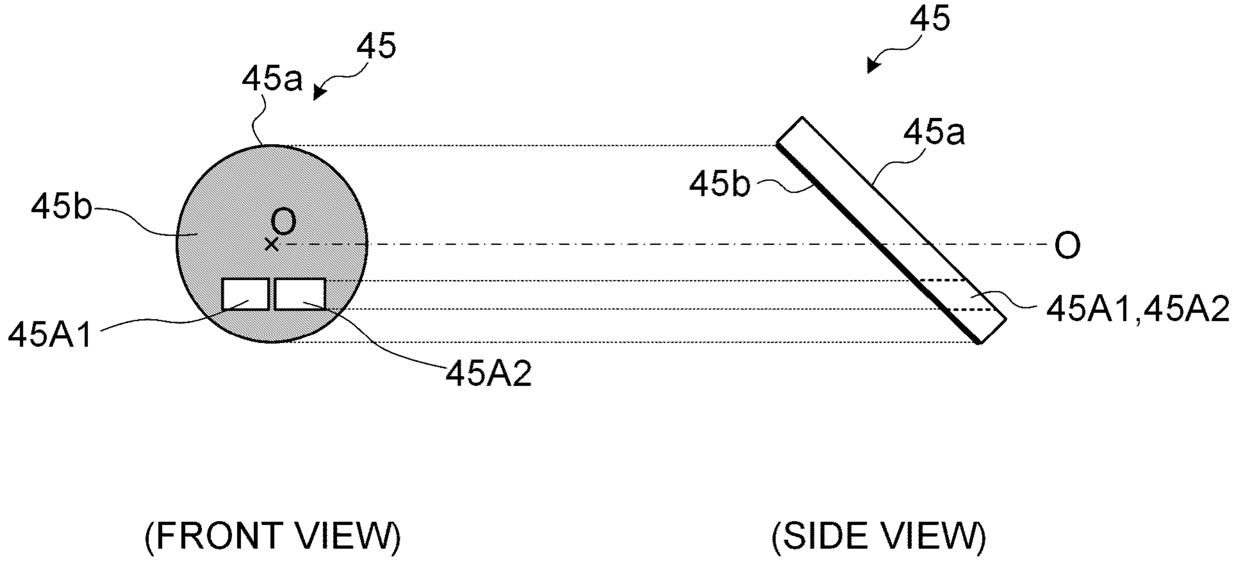


FIG. 17A

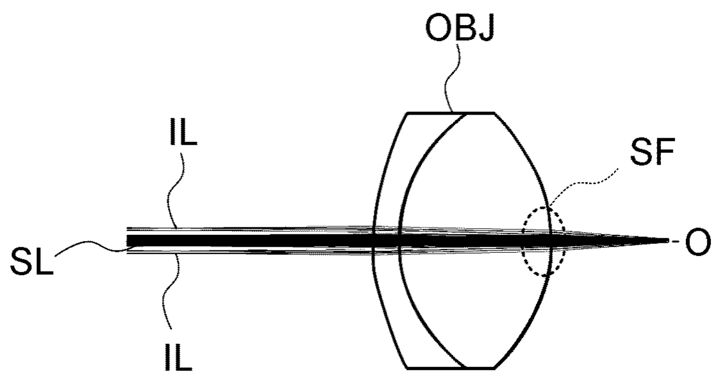

FIG. 17B

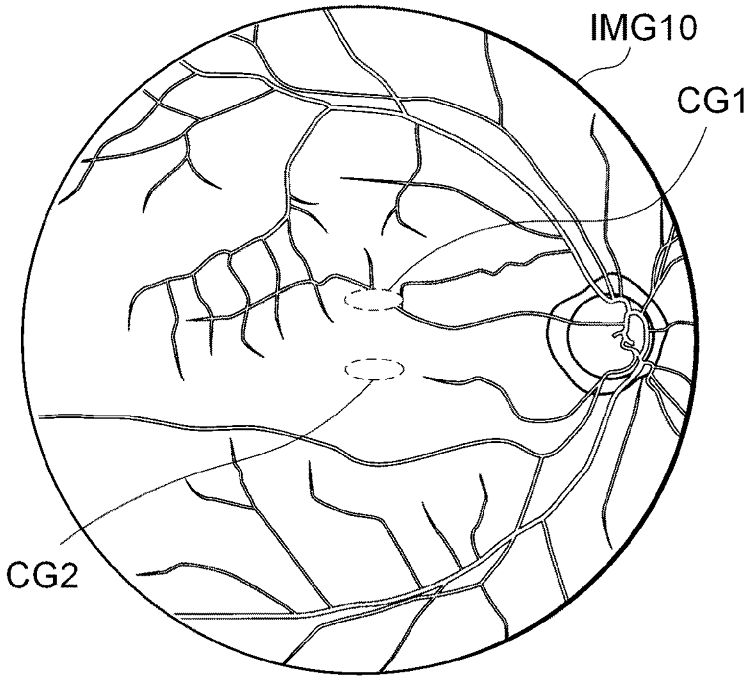


FIG. 17C

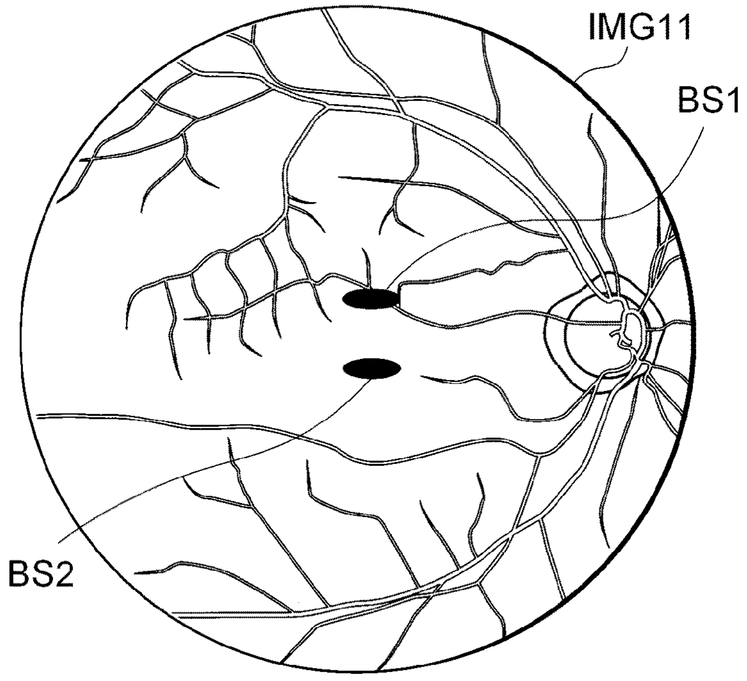

Europäisches Patentamt
European Patent Office
Office européen des brevets

# EUROPEAN SEARCH REPORT

Application Number
EP 25 21 1656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2023 043202 A (TOPCON CORP) 29 March 2023 (2023-03-29) * paragraphs [0038], [0041], [0044], [0047], [0050], [0051], [0053], [0057], [0069], [0070], [0271] * * figures 1,2A, 2C * ----- | 1,3,5,6, 8-15 | INV. A61B3/12 A61B3/14 A61B3/15 |
| Y | US 2023/014194 A1 (SHIMOSAWA RYOSUKE [JP] ET AL) 19 January 2023 (2023-01-19) * paragraphs [0035], [0037], [0043], [0052], [0060], [0062], [0069], [0076], [0077], [0080], [0130], [0162] * * figures 1, 4A, 4B, 12 * ----- | 1-15 | |
| Y | US 5 713 047 A (KOHAYAKAWA YOSHIMI [JP]) 27 January 1998 (1998-01-27) * column 3, line 50 - line 62 * * column 4, line 21 - line 32 * * figures 1A, 5, 7, 9 * ----- | 1-15 | |
| | | | TECHNICAL FIELDS SEARCHED (IPC) |
| A | US 2022/322935 A1 (SUZUKI MASAYA [JP] ET AL) 13 October 2022 (2022-10-13) * figures 1, 3 * ----- | 1-15 | A61B |

The present search report has been drawn up for all claims

1

| Place of search | Date of completion of the search | Examiner |
|---|---|---|
| Munich | 12 March 2026 | Neumann, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 21 1656

This annex lists the patent family members relating to the patent documents cited in the above-mentioned European search report. The members are as contained in the European Patent Office EDP file on
The European Patent Office is in no way liable for these particulars which are merely given for the purpose of information.

12-03-2026

| Patent document cited in search report | Publication date | Patent family member(s) | Publication date |
|---|---|---|---|
| JP 2023043202 A | 29-03-2023 | JP 7794589 B2 | 06-01-2026 |
| | | JP 2023043202 A | 29-03-2023 |
| US 2023014194 A1 | 19-01-2023 | CN 115297760 A | 04-11-2022 |
| | | EP 4122375 A1 | 25-01-2023 |
| | | JP 7469090 B2 | 16-04-2024 |
| | | JP 2021145896 A | 27-09-2021 |
| | | US 2023014194 A1 | 19-01-2023 |
| | | WO 2021187162 A1 | 23-09-2021 |
| US 5713047 A | 27-01-1998 | NONE | |
| US 2022322935 A1 | 13-10-2022 | CN 115003210 A | 02-09-2022 |
| | | EP 4094675 A1 | 30-11-2022 |
| | | EP 4413916 A2 | 14-08-2024 |
| | | JP 7414852 B2 | 16-01-2024 |
| | | JP 2023165819 A | 17-11-2023 |
| | | JP WO2021149280 A1 | 29-07-2021 |
| | | US 2022322935 A1 | 13-10-2022 |
| | | US 2025255483 A1 | 14-08-2025 |
| | | WO 2021149280 A1 | 29-07-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7831106 B
- US 8237835 B
- WO 2021205965 A
- JP 2020006172 A
- JP 61293430 A
- JP 2010259495 A
- JP 2013248376 A